# EUROPEAN PATENT SPECIFICATION

(11) **EP 3 681 487 B1**
(45) Date of publication and mention of the grant of the patent: **10.07.2024**
(21) Application number: 18855318.4
(22) Date of filing: 14.09.2018
(51) Int. Cl.: A61K 9/70, A61L 15/42, A61L 15/58, A61L 27/34, A61L 27/54, A61L 29/08, A61L 29/16, A61L 31/10, A61L 31/16, A61L 31/04, A61L 29/04, A61L 27/16

(54) **SELF ADHESIVE FILM FOR DELIVERY OF ACTIVES**
SELBSTKLEBENDE FOLIE ZUR ABGABE VON WIRKSTOFFEN
FILM AUTOADHÉSIF POUR L'ADMINISTRATION D'AGENTS ACTIFS

(30) Priority: 14.09.2017 US 201762558850 P
(43) Date of publication of application: 22.07.2020
(73) Proprietor: Allvivo Vascular, Inc., Lake Forest, California 92688 (US)
(72) Inventor: NEFF, Jennifer A., Lake Forest California 92688 (US); BAYRAMOV, Danir F., Lake Forest California 92688 (US)
(74) Representative: Maschio & Soames IP Ltd
(86) International application number: PCT/US2018/051087
(87) International publication number: WO 2019/055785

(56) References cited:
- EP-B1- 2 316 395
- WO-A1-00/06213
- WO-A1-2014/164027
- US-A1- 2011 056 609
- US-A1- 2016 074 559
- US-A1- 2016 144 067
- US-B1- 6 512 158

## Description

### FIELD OF THE INVENTION

The current invention relates to the field of accessory medical devices and accessory bioactive dosage forms designed to improve performance of primary medical devices and surgical procedures. The devices and methods described herein improve upon existing devices and methods by preventing infection development and alleviating complications associated with the primary device use and implementation of surgical or medical procedure and treatment of a patient.

### BACKGROUND

Various accessory or complimentary devices as well as accessory dosage forms capable of delivering antimicrobial and other bioactive agents in the vicinity of primary devices and surrounding body tissues are known. However, there is a need for improving performances of medical devices and reducing complications associated with development of infection and other undesired side effects during surgical procedures.

Attempts to solve the aforementioned need in the industry have been described, for example, US Pat. No. 9,486,560 B2 (F. Buevich), describes polymer coated surgical meshes for use with cardiac rhythm management (CRM) devices. US Pat. No. 9,295,809 B2 (K.W. Sheetz) describes a mesh sleeve for use with access ports and catheters. US No. 8,042,544 B2 (K.R. Ward et al) describes a removable water absorbing sponge material is designed to be placed above the cuff (balloon) of an endotracheal tube (ETT) during patient intubation. US Pat. No. 9,480,643 B2 (A.J. Tipton) describes an implantable solid tacky (adhesive) polymer film having a releasable bioactive agents that is capable of adhering itself or through an adhesive to a substrate. US Pat. No. 9,247,736 B2 (K.M. Ylitalo) describes a multi-layer film in which a first layer comprises adhesive and a second layer (on an opposite side than the first layer) comprises an antimicrobial film. US 2016/074559 discloses a biodegradable covering for a medical implant, the covering comprising a highly plasticized gelatin and a drug.

Known accessory devices include delivery of bioactive agents during surgical procedures and/or device insertion. However, known devices are limited in their application to a wide variety of devices, in that each device has to be tailored to the individual specifications of an individual medical device and/or procedure, such as vascular catheters, arteriovenous grafts, arteriovenous fistulas, urinary catheters and stents, suprapubic catheters, tracheal, endotracheal and tracheostomy tubes, esophagostomy tubes, trachea-esophageal voice prosthesis, peritoneal dialysis catheters, surgical drainage tubes, etc. Further, although known accessory devices disclose various adhesives for adhering to various substrates, these devices are limited in their application to a wide variety of substrates and/or adhering to body tissues. Therefore, there is a need in the art for an accessory device which can be applied to a wide variety of devices in varying shapes/sizes, which also delivers bioactives and is capable of adhering to substrates as well as body tissues.

### SUMMARY

According to one embodiment of the invention, a film for self-adhering to a surface of a three dimensional object and delivery of one or more bioactive agents within a body is provided.

The film according to claim 1 comprises one or more layers, where the film and each film layer has a first side and an opposite second side;
a width, having a width dimension;a first end portion and a second end portion, the first and second end portion positioned at opposite ends of the length of the film;
a length, having a length dimension which is of sufficient length such that the film wraps at least once around an outer surface of the three-dimensional object with the first and second end portions at least partially overlapping;a plurality of component regions positioned on, or within, or partially with, or a combination thereof, the first and second sides of the film layers, the plurality of component regions selected from the group consist of:one or more a carrier regions, each carrier region, individually, comprising one or more bioactive agents, the one or more bioactive agents being the same or different,
wherein the bioactive agent is releasable from the one or more carrier regions when the film is wrapped around the three-dimensional object when the film is positioned within a body;
one or more adhesive regions, each adhesive region extending at least a portion of the width of the film; and
one or more blank regions which does not contain adhesive or bioactive agent, wherein:
   the first side of the film comprises at least a first component region and a second component region, which are the same or different, wherein at least one of the first and second component regions is a carrier region, and
   the second side of the film comprises (i) at least a first adhesive region positioned on the first or the second end portion of the film, the first adhesive region being capable of self-adhering when the film engages at the opposite end portion of the film when the film is wrapped at least once around said three-dimensional object; and (ii) one or more blank regions or one or more carrier regions.

According to the present invention, in one embodiment, a self-adhesive film for covering at least a portion of a surface of a three-dimensional (3D) object such as a primary medical device or body tissue with the purpose of delivering bioactive agents, such as antimicrobials, drugs, and other biologically active agents in the vicinity of the medical device and adjacent tissue is provided. The film is configured to self-adhere when engaging another portion of the self-adhesive film after wrapping around at least a portion of a surface of a 3D object and creating an adhesive bond independent of the surface properties of the 3D object.

Various types of bioactive agents can be used in the self-adhesive film described herein to impart biological activity leading to treatment, cure, preventing, mitigating, ameliorating, diagnosing or other favorable effect on a medical condition, disease, disorder, infection, and the like. Examples of bioactive agents that can be incorporated into the self-adhesive film herein include, but are not limited to, synthetic drugs comprising low- and high- molecular weight organic compounds, biologically derived bio- polymers and oligomers and their synthetic and engineered analogues such as proteins and peptides, lipoproteins and lipopeptides, DNA, RNA, iRNA, nucleic acids, antibodies, antibody fragments, antigenic compounds, enzymes, hormones, polysaccharides, lipopolysaccharides, lipids, surfactants and the like.

Bioactive agents considered for use in the self-adhesive film include but are not limited to antibiotics, antimicrobial agents, anti-biofilm agents, antifungals, antivirals, anti-scarring agents, anticoagulants, anti-thrombotic agents, cardiovascular agents, hormones, growth hormones, tissue growth agents, thyroid and anti.-thyroid agents, anti-inflammatory agents, immunomodulating agents, anti-histamines, anesthetics, analgesics, anorexics, antacids, antiarthritics, anabolics, antimetabolite agents, antinauseants, anti-emetics, anti-asthmatic agents, anticholesterolemic agents, antipyretic, anti-manic agents, anti-diarrheals, diuretics, antineoplastic agents, anticancer agents, anti-obesity agents, laxatives, neuromuscular agents, vasodilators, bronchiodilators, coronary dilators, cerebral dilators, uterine relaxants, antispasmodics, antitussive agents, expectorants, mucolytic agents decongestants, gastrointestinal sedatives, antiulcer agents, appetite suppressants, anti-convulsants, psychotropics, antidepressants, sedatives, tranquilizers, hypnotics, anti-diabetic agents, vitamins, mineral supplements, stimulants, diagnostic agents.

According to one embodiment, the self-adhesive film wraps at least once around the surface of the three-dimensional object with the first and second end portions of the film at least partially overlapping, and when at least one adhesive region, the adhesive region being positioned at an end portion of the self-adhesive film, is configured to self-adhere when engaging another portion of the self-adhesive film or tire three-dimensional object.

According to another embodiment, the self-adhesive film comprises one or more carrier regions positioned on the first side of the film, on the second side of the film, or both sides of the film, each carrier region comprising one or more bioactive agents, the one or more bioactive agents being the same or different. The bioactive agents are delivered with rates that are controlled independently.

According to another embodiment, the self-adhesive film comprises a plurality of blank regions, each blank region comprising a layer which does not contain a bioactive agent. Each blank region is independently positioned on the first side of the film, on the second side of the film, or the both sides of the film. The one or more blank regions are positioned adjacent or in-between the one or more bioactive carrier regions, with the positioning configured such that the controlled release mode of a bioactive is realized. According to this embodiment, the controlled release is characterized by delayed or slower release of the bioactive agent from the carrier region which is positioned beneath the blank region, as compared to a carrier region which is not positioned beneath a blank layer.

Yet in the other various invention embodiments, the self-adhesive film comprises a length dimension of sufficient length to wrap around the surface of the three-dimensional object at least twice to form at least two overlapping layers, wherein the film and the one or more adhesive regions are configured such that at least one of the adhesive regions self-adheres to the film or the surface of the three-dimensional object.

According to other embodiments, the film has a length which is sufficient to wrap around the surface of the three-dimensional object at least twice, in a two layer film wrap. In one embodiment, the film is a two-layer film wrap, where at least one of a plurality of carrier regions is positioned on the film such that it is directly atop another of the plurality of carrier regions. One of the carrier regions may define, at least in part, an outermost layer of the film. In this embodiment, the controlled release of the bioactive is controlled by the overlapping carrier regions.

As described herein, there are a plurality of combinations of carrier, blank, and adhesive regions on the film. Twenty-five (25) positioning combinations of the carrier, blank, and adhesive regions are described herein which can be used to achieve a desired bioactive release mode. In a two layer film wrap which has a carrier region positioned in at least only one film layer and at least only on one side of the layer, one of 45 positioning combinations of the carrier, blank and adhesive regions can be used to achieve desired bioactive release mode. The combinations of carrier, blank, and adhesive regions on the film are increased as the layers of film increase. Accordingly, the film can be modified and fine-tuned to a variety of desired bioactive release rates, with a plurality of bioactives being delivered within a body.

According to another embodiment a device, tissue or anatomical structure having a self-adhesive film according to claim 1 for delivery of one or more bioactive agents within a body is provided. The device, tissue, or anatomical structure comprises an element having an outer surface and a substantially three-dimensional shape and a self-adhesive according to claim 1. The multi-layer composite structure comprises a first side, an opposite second side, a carrier layer comprising one or more bioactive agents, and at least one adhesive region, the adhesive region being positioned at an end portion of the self-adhesive film, wherein the adhesive region is configured to self-adhere when engaging another portion of the self-adhesive film or the device when wrapped one or more times around the outer surface of the device.

A method, not part of the invention, of delivering one or more bioactive agents within a body is provided. The method comprises providing a three-dimensional object having an outer surface and providing a self-adhesive film according to the present invention. A self-adhesive film is wrapped around the outer surface of die three-dimensional object such that the adhesive region of the self-adhesive film self-adheres when engaging another portion of the self-adhesive film or the three-dimensional object when wrapped one or more times around the outer surface of the three-dimensional object. One or more bioactive agents is then delivered within the body. When the three-dimensional structure is a medical device, the self-adhesive film is wrapped around the medical device and self-adhesive wrap is positioned within the body for delivery of the one or more bioactive agents within the body. When the three-dimensional structure is tissue or an anatomical structure, the self-adhesive film is wrapped around the tissue or the anatomical structure for delivery of the one or more bioactive agents within the body.

According to the embodiments described herein the one or more bioactive agents can be delivered to the three-dimensional structure, or from the self-adhesive film to the body, or to both the three-dimensional structure and the body.

### BRIEF DESCRIPTION OF THE DRAWINGS

These and other features, aspects and advantages of the present invention will become better understood from the following description, appended claims, and accompanying figures where:
**Figure 1** is a side view showing a general schematic for a film positioned around a three-dimensional (3D) object, according to the present invention;
**Figure 2** is a schematic side view of a film positioned around a 3D object, showing the positioning of a component on a film, according to another embodiment of the present invention.
**Figure 3** is a representation of positioning combinations of components in a film segment and corresponding modes of bioactive release: A-adhesive; C - carrier; B (F) - blank; F (equivalent to B) - first film region that does not have adhesive and carrier, according to another embodiment of the invention;
**Figure 4** is a schematic side view of a film positioned around a 3D object, showing representations of possible positioning combinations of components in a film segment: A-adhesive; C - carrier; B (F) -blank; F (equivalent to B) - first film region that does not have adhesive and carrier, according to another embodiment of the invention;
**Figure 5** is a schematic side view of various films wrapped around a 3D object showing examples of positioning of adhesive regions in a film for creating self-adhesive bond, according to other embodiments of the invention;
**Figure 6** is a schematic side view of various films wrapped around a 3D object, showing examples of separation of multiple carrier regions in a film; C1, C2, C3 - individual carriers, B-blank region. A- adhesive region, according to other embodiments of the invention;
**Figure 7** is a schematic side view of various films wrapped around a 3D object, showing examples of positioning of blank regions and carrier regions to regulate bioactive agent release modes, according to other embodiments of the invention;
**Figure 8** is a side view showing a schematic representation of a film with a length sufficient to wrap around the surface of a 3D object at least twice, according to another embodiment of the invention;
**Figure 9** is a schematic side view of various films wrapped around a 3D object, showing examples of positioning adhesive regions in a film, according to the present invention, with a length sufficient to wrap around the surface of a 3D object at least twice. Wherein one or more adhesive regions are configured to self-adhere to the film or the surface of the three-dimensional object, according to other embodiments of the invention;
**Figure 10** is a schematic side view of various films wrapped around a 3D object, showing examples of positioning carrier regions (C), blank regions (B) and adhesive regions (A) in a two layered wrap of a film, according to the present invention, to realize various modes of bioactive release, according to other embodiments of the invention;
**Figure 11** shows examples of positioning combinations for carrier regions (C), blank regions (B) and adhesive regions (A) in a two layered wrap of a film, according to the present invention, leading to various modes of bioactive release, according to another embodiment of the invention;
**Figure 12** is a schematic top perspective view of an exemplary film, showing an example of an antimicrobial carrier member, where **C**- AMP carrier; **A-** adhesive; **D-** removable polyester release liner and **E**- removable paper carrier, according to another embodiment of the invention;
**Figure 13** is a schematic top perspective view of an exemplary film, showing an adhesive member on a mesh. **A**- Mesh regions impregnated with adhesive. **B**- mesh region not impregnated, according to another embodiment of the invention;
**Figure 14** is a schematic top perspective view of an exemplary film, showing two active carrier regions. **C1**- mesh region impregnated with antimicrobial, **C2-** mesh region impregnated with anesthsetic, **A-** Mesh regions impregnated with adhesive, according to another embodiment of the invention;
**Figure 15** is a schematic top perspective view of an exemplary film, showing two active carrier regions. **C1**- antimicrobial carrier region, **C2-** anesthsetic carrier region, **A**- mesh impregnated with adhesive, according to another embodiment of the invention;
**Figure 16** is a schematic top perspective view of an exemplary film, showing two actives and four active carrier regions. **C1**- antimicrobial carrier on mesh, **C2-** anesthsetic carrier on mesh, **A-** adhesive on mesh, according to another embodiment of the invention;
**Figure 17** is a schematic top perspective view of an exemplary film, showing two actives and eight active carrier regions, where **C1**- antimicrobial carrier regions; **C2** - anesthetic carrier regions; **A** - adhesive sheet; and **B** - polyester mesh, according to another embodiment of the invention;
**Figure 18** is a schematic top view of a regions pattern on a film, suitable for wrapping medical tube with 9.5 mm OD. **A, C,** and **E** are designated for adhesive regions, **B1, B2, D1**, and **D2** are designated for actives and blank regions, according to another embodiment of the invention; and
**Figure 19** is a schematic top view for wrap designs of a film, where **A, C,** and **E** are adhesive regions in all four designs, according to other embodiments of the invention.
**Figure 20** is a schematic top view of self-adhesive films designed for wrapping endotracheal tubes, according to another embodiment of the invention.
**Figure 21** is a plot of ASP1 antimicrobial peptide release from ETT wraps SAF-1, SAF-2 and SAF-3 (n=3) shown in Figure 20.
**Figure 22** is a plot of actives release from ETT wraps SAF-4, SAF-5 and SAF-6 (n=3) shown in Figure 20.
**Figure 23** is a schematic top view of self-adhesive films designed for wrapping 19Fr wound drain tubes with the top facing device surface, according to another embodiment of the invention.
**Figure 24** is a plot of ASP1 antimicrobial peptide release from wound drain tube wraps SAF-7, SAF-8 and SAF-9 (n=3) shown in Figure 23.

### DETAILED DESCRIPTION

According to the present invention, a film for self-adhering to a surface of a three dimensional object and delivery of one or more bioactive agents within a body is provided. The film is advantageous over the known films in the art, in that the film is self-adhering to both the surface of a 3D object, and/or a tissue within a body. The film according to the present invention can be applied to a wide variety of devices in varying shapes/sizes and also delivers bioactives. In a multi-layer film according to the invention, the bioactives can be delivered in a body in a controlled release, with the release of the bioactive being controlled by the layering of the film on the object or tissue. A significant advantage of the film according to the invention is that as the same film can be configured and used on a wide variety of devices, tissues, and anatomical structures, which lowers the regulatory hurdle for commercial implementation of the device, as a separate film will not need regulatory approval for each device to which it is applied.

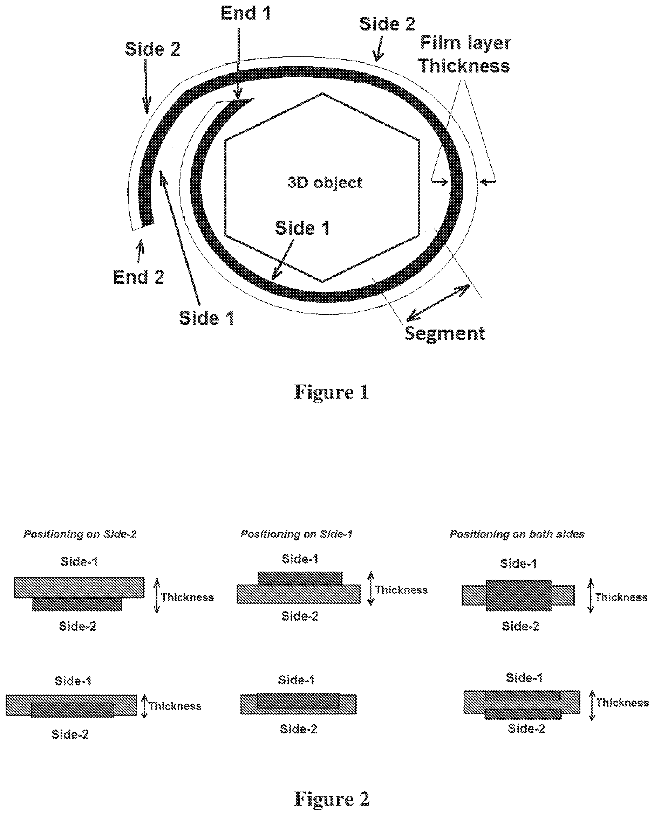

Referring now to Figure 1, a general schematic of a side view of a film, according to one embodiment of the invention, positioned around an object is shown. As shown in Figure 1, the film has an elongated form comprising a length dimension, a width dimension, and a thickness dimension, according to one embodiment of the invention. It will be understood by those of skill in the art that the thickness is associated with the film layer and can vary depending on location. It will also be understood that the film length dimension could be either longer or shorter than the width dimension, and the assignment of the length dimension could be purely based on convenience, such as considerations associated with the direction of film application on a 3D object and/or the order of film application.

The film, also referred to herein as a wrap, is positioned on tissues or anatomical structures including nerves, vessels, connective tissues including tendons, ligaments and bones, esophagus, intestine, colon, surgical anastomoses and fistulas (such as arteriovenous fistulas), for example. The wrap can also be positioned on a medical device including catheters, vascular catheters, vascular grafts, arteriovenous grafts, urinary catheters, urinary stents, suprapubic catheters, tracheal tubes, such as endotracheal or tracheostomy tubes, esophagostomy tubes, trachea-esophageal voice prosthesis, peritoneal dialysis catheters, drainage tube/catheters, tympanostomy tubes, orthopedic implants, orthopedic fixation devices, pace makers, heart assist devices, neurostimulation devices, drug delivery devices, or feeding tubes.

As used herein, the following terms have the following meanings.

The term three-dimensional object (3D object) refers to a medical device, a portion of medical device, a body part, tissue, and/or anatomical structure, or a combination of a medical device, tissue and/or anatomical structure such as a fixation device positioned on or next to a body part or tissue on which the self-adhesive film is intended to be applied or applied.

The term "adhesive", also referred to herein as "adhesive component" or "adhesive region" refers to a material capable of sticking to itself or another material to form an adhesive bond. It is known to those skilled in the art that many types of adhesives exist such as pressure sensitive adhesives, light curable adhesives, moisture curable adhesives, heat curable adhesives, and the like. Therefore, it will be understood that the term "adhesive" used herein is not intended to be limited to any particular type of adhesive.

The term "bioactive agent", also referred to herein as "bioactive", "biologically active" or "active" refers to a medicament or agent that leads to treatment, cure, preventing, mitigating, ameliorating, diagnosing or other favorable effect on a medical condition, disease, disorder, infection, and the like.

The term "blank", also referred to herein as "blank component" or "blank region" refers to a region of the film that does not have adhesive and bioactive. It will be understood that the blank can comprise any material that does not have adhesive and/or bioactive carrier. In one embodiment blank is referred to a region comprising the first film or a film component that does not have adhesive and bioactive carrier.

The term "carrier", also referred to herein as "carrier component" or "carrier region" or "bioactive carrier" refers to a pharmaceutical composition comprising one or more bioactive agents.

The term "components" refers to constituents imparting functional features such as adhesive properties, bioactive delivery mode, structural integrity as well as other aspects associated with practical conveniences and considerations for exercising current invention such as methods and ease of film fabrication. The film comprises "components" that form "regions", also referred to as "layers". It will be understood the term "component" is not intended to be limiting to particular materials, compositions, adhesives, bioactive agents but is being used herein for the purpose of describing the invention embodiments.

The term "first film", also referred to herein as "the first film component" or "a film component" refers to a film onto which other components could be positioned to fabricate or otherwise create by any means the self-adhesive film described herein. In one embodiment, the first film or the film component is a non-porous flexible film, such as a polymer film, a metal foil, a composite material, a paper, and the like. In another embodiment, the first film is a porous flexible film such as a mesh, a non-woven or woven fabric, a sponge, foam, and the like. Yet, in another embodiment the first film comprises an adhesive component. Yet, in another embodiment the first film comprises a bioactive carrier.

The terms "first end" and "second end", as used herein, the film has two ends: the first end and the second end. The terms the first end and the second end can be explicitly based on convenience choices, such as considerations associated with the direction of film application on a 3D object and/or the order of film ends application on a 3D object. For example, the term "first end" might be assigned to the end that is applied first on the 3D object and the term "second end" to the opposite end intended to be applied last on the 3D object.

The terms "first side" and "second side", as used herein, the film has two sides: the first side and the second side. For the convenience of describing specific invention embodiments, the term "the first side" is used to refer to the side facing towards the 3D object and the term "second side" is used to refer to the opposite side facing outwards from the 3D object.

The term "layer" when referring to a film, will be understood as a space occupied by the full thickness of the film from the first side to the second side of the film. However, when the term "layer" refers to a component region it will be also understood here as the space occupied by the component region in the film. In one embodiment, a component layer occupies a portion of the full film thickness from one side. Yet in another embodiment a layer of a component region occupies the full thickness of the film from the first side to the second side of the film. As also described herein, a "layer" has a plurality of regions which may be comprised of separate and distinct materials, adhered, or otherwise bonded to each other, or the regions on the layers may be comprised of a single material which is impregnated with another agent, such as an adhesive or bioactive to form the region on the film, or a combination thereof.

The term "positioning" or "positioned" refers to a location of a component on a film, and associated with creating a component region on a film by applying, dispensing, printing, spraying, adhering, laminating, fixing, or by other means forming a region of a component on a film. In one embodiment "positioning" refers to forming a region of a component on the first film component. In another embodiment "positioning" refers to a region on a film that does not have adhesive or bioactive carrier.

The term "segment" of a film refers to a film portion comprising a length dimension, a width dimension, and a thickness dimension. A segment has the first side facing towards the 3D object and the second side facing outside the 3D object.

The term "substrate" refers to a material to which adhesive can be applied to form an adhesive bond of desired strength.

The term "stack", when referring to film layers or film segments, will be understood here as a construct arising when layers of film overlap and are positioned on top of each other.

According to the present invention, the film for self-adhering to a surface of a three dimensional object and delivery of one or more bioactive agents comprises one or more layers, a length dimension, a width dimension (also referred to herein as a thickness), a first side and an opposite second side, a first end portion and a second end portion. The first and second end portions are positioned at opposite ends of the length of the film. Referring again to Figure 1, the length dimension of the film (shown in Figure 1 as the distance from End 1 to End 2) is of sufficient length such that the film wraps at least once around the surface of the three-dimensional object with the first and second end portions at least partially overlapping.

Referring now to Figure 2., a side view of a film, or first film, having one or more components, such as a bioactive or adhesive, positioned on the film in the component region is shown. The components are positioned in various configurations, according to the embodiment of the invention as shown in Figure 2. As shown in Figure 2, the film, or first film is shown as a grey segment. The one or more components positioned on the film in the component region is shown as a black segment. As will be understood by those of skill in the art, the positioning of a component could be on one side, on the other side or on both sides of a film as it is shown in schematically in Figure 2. Positioning of a component on a film may be associated with a change in the film segment thickness as compared to other film segments. As shown in Figure 2, according to one embodiment, a component region occupies the whole thickness of the film. In another embodiment, the component region occupies a portion of film thickness from one side of the film. Yet in another embodiment the component region occupies a portion of a film thickness from both sides of the film, as shown in schematically in Figure 2.

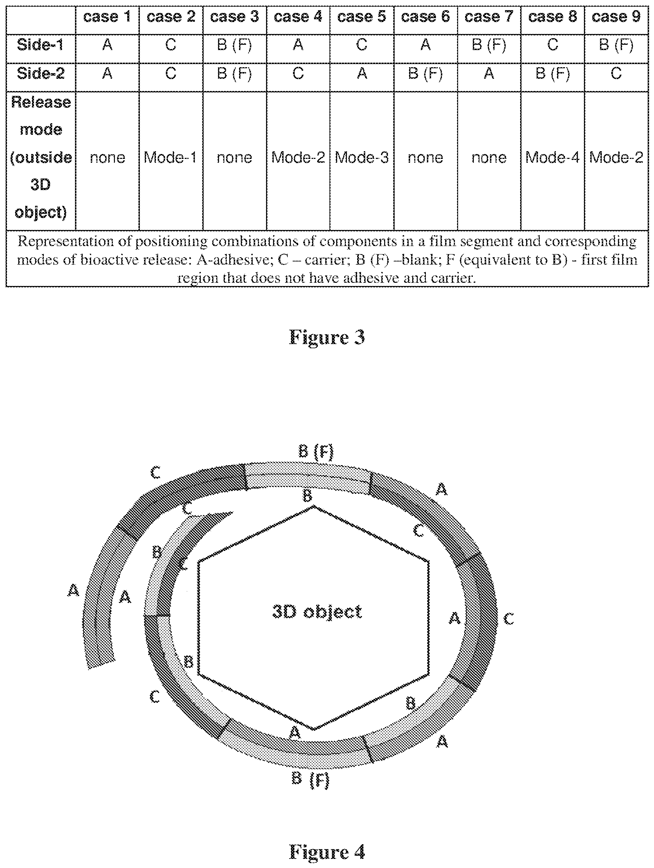

Referring now to Figure 3, a table representing various positioning combinations of components in a film segment and corresponding modes of bioactive release is shown where: A represents adhesive; C represents carrier; B (F) represents blank; F (equivalent to B), a first film region that does not have adhesive and carrier. Referring now to Figure 4, a schematic side view representation of possible positioning combinations of components in a film segment is shown where: A represents adhesive; C represents carrier; B (F) represents blank; F (equivalent to B'), a first film region that does not have adhesive and carrier. As will be also understood by those of skill in the art, positioning of adhesive, carrier, and blank components on two (2) sides of a film segment can lead to 9 possible configurations of a film segment as well as to 4 different modes of bioactive release as shown in table form in Figure 3 and schematically in Figure 4.

As will be understood by those of skill in the art, adhesion of an adhesive film to a substrate, for example a surface of a 3D object depends both on the physico-chemical properties of the adhesive and the nature of the substrate. As will also be understood to those of skill in the art, the same adhesive composite may not develop adhesive bond of the same desired strength with substrates made of substantially different materials such as, for example, metal, polyethylene, silicone rubber or body tissue. In addition, many devices are produced using mold release agents or have coatings applied to their surfaces such that they are not conducive to binding well to an adhesive.

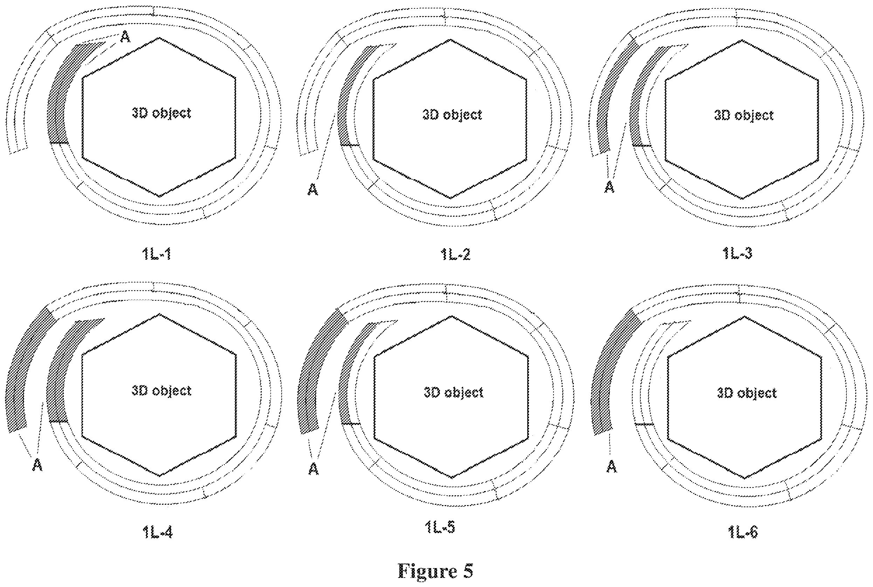

Referring now to Figure 5, a side view of a film, according to other embodiments is shown, showing examples of positioning of adhesive regions (A) in a film for creating a self-adhesive bond. As described herein, the film is capable of self-adherence and adhesive regions (A) in one or both of: (i) a first adhesive region positioned on the first side of the film and positioned at the second end portion of the film, and (ii) a second adhesive region positioned on the second side of the film and positioned at the first end portion of the film. As shown in Figure 5, the film and the adhesive region are configured such that one of the first adhesive region or the second adhesive region self-adheres, when engaging the film at the opposite end portion of the film, or the surface of the three-dimensional object when the film is wrapped at least once around the three-dimensional object.

One advantage of the invention is that when a self-adhesive film wraps at least once around the surface of the three-dimensional object with the first and second end portions at least partially overlapping, and when at least one adhesive region, the adhesive region being positioned at an end portion of the self-adhesive film and is configured to self-adhere when engaging another portion of the self-adhesive film or the device, a consistent adhesive bond between the overlapping layers of the self-adhesive film is created independent of the substrate material of the 3D object surface. Accordingly, in the regions where the self-adhesive film overlaps it serves both as adhesive and substrate thus creating an attachment bond, where the strength of the bond does not depend on the type of material used to form the medical device or does not depend on adhesion to a body tissue as is demonstrated in schematic examples in Figure 5.

As is known to those skilled in the art, the feasibility of incorporating different bioactive agents into a single composite material, a carrier, depends on their chemical compatibility and may be limited by the chemical nature of the bioactives as well as the chemical nature of the carrier material. Unfavorable physico-chemical interactions (incompatibility) may occur both between the bioactive agents as well as between the bioactives and the carrier material. Such incompatibility in turn may alter stability and release rates of the bioactive agents. Unfavorable interactions that lead to incompatibility may include such processes as chemical reactions, complexation, formation of poorly soluble salts, and the like. The carrier composition or formulation suitable for incorporating one bioactive agent may not be suitable or optimal for incorporating a second bioactive to maintain its activity, potency, stability and desired bioactive release mode. Thus, according to the current invention, separation of individual carriers into different carrier regions allows to avoid incompatibility risks and optimize performance properties, such as stability, dosing and duration of bioactive agent's release.

The film according to the present invention has one or more a carrier regions, each carrier region, individually, having one or more bioactive agents. The one or more bioactive agents can be the same or different, and each of the one or more carrier regions, individually, is positioned on one or both of the first and second sides of the film. The film may optionally have one or more blank regions, each blank region comprising a layer which does not contain adhesive or a bioactive agent. The one or more blank regions are positioned on the first side or the second side of the film layer and in-between or adjacent to one of the plurality of carrier regions or the adhesive regions.

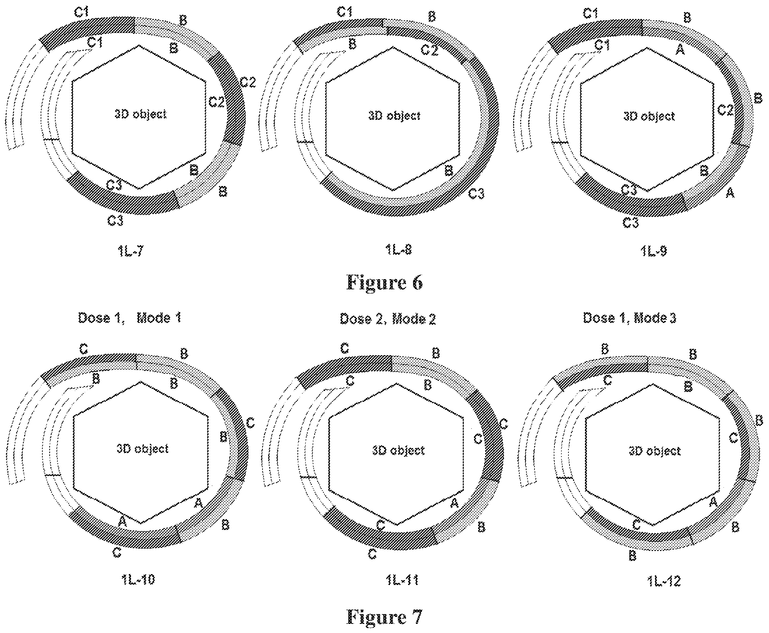

Referring now to Figure 6, a schematic side view of a film showing examples of separation of multiple carrier regions in a film is shown, where C1, C2, and C3 represent individual carriers; B represents a blank region; and A represents an adhesive region. As shown in Figure 6, the separation of individual carriers in a film, as is described herein, is achieved by positioning one or more blank regions, a plurality of blank regions, each blank region comprising a layer which does not contain adhesive or a bioactive agent, the one or more blank regions being positioned on the first side or the second side of the film layer and in-between or adjacent to one of the plurality of carrier regions or the adhesive regions as it is demonstrated in the example schematics in Figure 6.

Referring now to Figure 7, a side view of examples of various films positioned around a 3D object is shown schematically, where the film is formed having areas of blank regions (B) and carrier regions (C) which can regulate bioactive agent release modes. As will be understood by those of skill in the art, the bioactive release mode, the release duration and the time course of the release rate, apart from the nature of the bioactive agent and the carrier material, depend on the total amount of the active present in the carrier, thickness of the carrier and the presence of diffusion barrier layers acting as a membrane on the surface of the carrier region from which the bioactive is released. Accordingly, in one embodiment of the invention, one type of bioactive release mode is realized when a carrier region is a layer positioned on the second side of the film and defines at least in part an outermost layer of the film, as shown in Figure? in the schematic 1L-10 (Mode 1). In another embodiment, another type of bioactive release mode is realized when a carrier region is a layer positioned on both sides of the film and defines at least in part an outermost layer of the film as it is shown in Figure7 in the schematic 1L-11 (Mode 2). Yet, in another invention embodiment a blank region acts as a regulator for release mode of the bioactive agent, when at least one of the plurality of blank regions is positioned on the film such that it is directly atop one of the plurality of carrier regions when the film is wrapped around the three-dimensional object as it is shown in Figure7 in the schematic 1L-12 (Mode 3).

According to another embodiment, the film and the one or more adhesive regions may be configured such that at least one of the adhesive regions self-adheres to the film or the surface of the three-dimensional object when the film is wrapped at least twice around the three-dimensional object. The bioactive agent is released from the carrier region of the film when the film is wrapped around the three-dimensional object and positioned within a body. According to one embodiment, the film has a length dimension which is sufficient to wrap around the surface of the three-dimensional object at least twice to form at least two overlapping layers. At least one adhesive region is positioned at the end portion of the film to self-adhere to the underlying film layer to form an overlapping multilayer film structure.

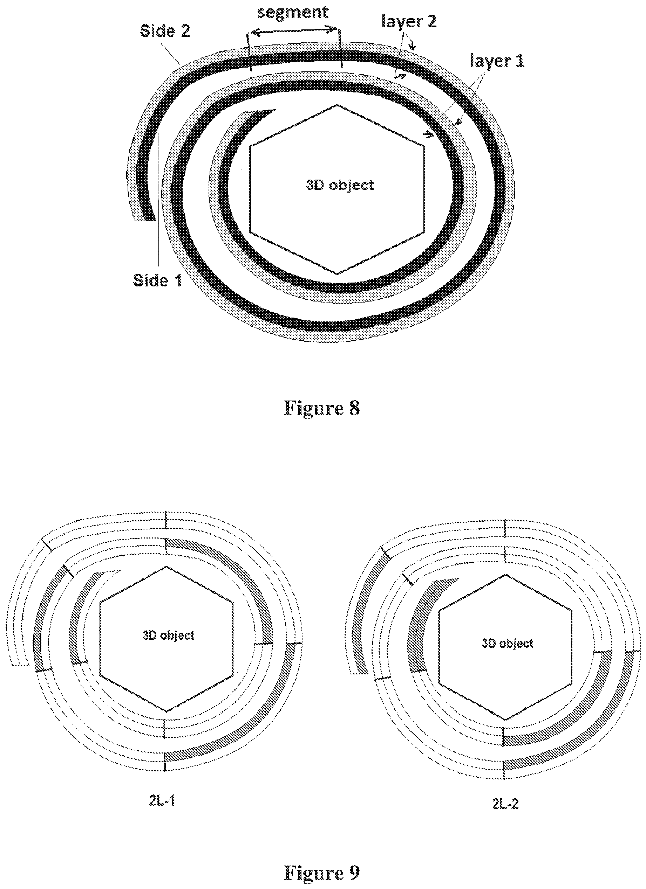

Referring now to Figure 8, a side view of an exemplary self-adhesive film with a length sufficient to wrap around the surface of a 3D object at least twice is shown schematically. According to the embodiment shown in Figure 8, the self-adhesive film comprises a length dimension of sufficient length to wrap around the surface of the three-dimensional object at least twice to form at least two overlapping layers. Referring now to Figure 9, a side view of various exemplary self-adhesive films having adhesive regions positioned to self-adhere to the film or the surface of a 3D object is shown schematically. As shown in Figure 8 and Figure 9, the film and the one or more adhesive regions are configured such that at least one of the adhesive regions self-adheres to the film or the surface of the three-dimensional object when the film is wrapped at least twice around the three-dimensional object. As shown in exemplary Figure 9, the adhesive regions are configured to self-adhere to the film or the surface of the three-dimensional object. Side view.as demonstrated in schematics in Figure 9.

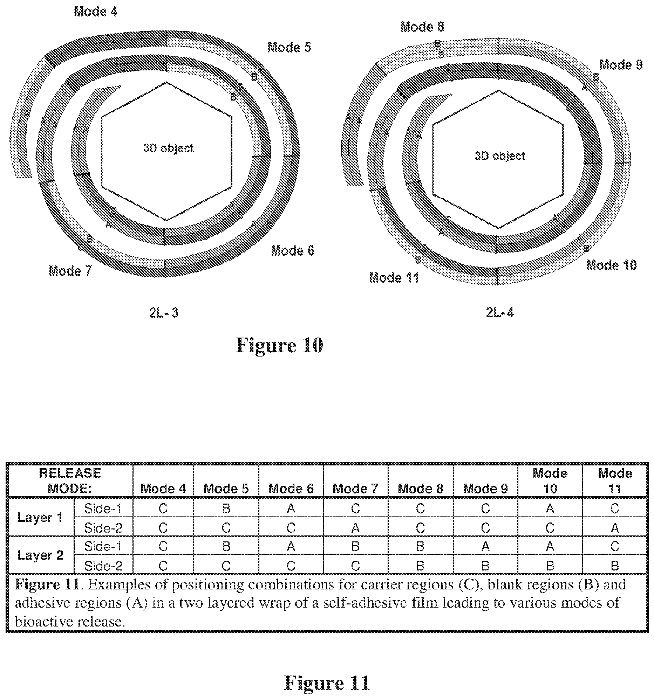

Referring now to Figure 10, a side view of various exemplary self-adhesive films positioned around a 3D object is shown schematically. According to this embodiment, the self-adhesive films have carrier regions (C), blank regions (B), and adhesive regions (A) positioned in a two layered wrap to realize various modes of bioactive release. According to this embodiment, when a self-adhesive film comprises a length dimension of sufficient length to wrap around the surface of the three-dimensional object at least twice, various modes of active release are achieved when two segments of the two film layers are stacked. In one embodiment, one mode of bioactive release occurs when at least one of the plurality of carrier regions is positioned on the film such that it is directly atop another of the plurality of carrier regions and at least one carrier region is a layer positioned on the second side of the film and defines least in part an outermost layer of the film (*see,* Figure 10, schematic 2L-3, Mode 4). In another embodiment, when the film length is sufficient to wrap around the surface of the three-dimensional object at least twice, and at least one carrier region is a layer positioned on the second side of the film outer layer and defines least in part an outermost layer of the film, different modes of bioactive release occur when blank and/or adhesive regions are positioned atop of inner carrier regions in the film wrap and serve as release regulators as it is demonstrated in Figure 10, schematic 2L-3, Modes 5,6 and 7. In a preferred embodiment, at least one of a plurality of carrier regions is positioned on the film such that it is directly atop another of a plurality of carrier regions when the film is wrapped around the three-dimensional object. According to another preferred embodiment, at least one of a plurality of blank regions is positioned on the film such that it is directly atop one of a plurality of carrier regions when the film is wrapped around the three-dimensional object and the blank region acts as a regulator for release of the bioactive agent. In yet another preferred embodiment, at least one of a plurality of blank regions acts as a regulator for release of the bioactive agent when the film is wrapped around the three-dimensional object.

According to other embodiments, when the film length is sufficient to wrap around the surface of the three-dimensional object at least twice, the other different modes of bioactive release occur when at least one carrier region is positioned in an inner layer of the film wrap, the blank regions are positioned on the second side of the film outer layer and positions of blank and/or adhesive regions in the inner layer of the film are configured to serve as release regulators as it is demonstrated in Figure 10., schematic 2L-4, Modes 8,9,10 and 11. Referring now to Figure 11, examples of positioning combinations for carrier regions (C), blank regions (B) and adhesive regions (A) in a two layered wrap of a self-adhesive film leading to various modes of bioactive release is shown in table form. The examples of positioning combinations shown in Figure 10 are also summarized in table form in Figure 11. As will be understood by those of skill in the art, the examples of positioning the carrier, adhesive and blank regions described in Figures 10 and 11 are for demonstration purposes only and do not cover all possible combinations of the regions to achieve desired mode of active release contemplated by the invention. For example, shown in Figure 3 there are in total 9 possible positioning combinations of carrier, adhesive and blank in a film layer, and amongst these 9 combinations there are 5 positioning combinations utilizing the carrier regions (cases 2,4,5,8 and 9 in Figure3). Thus, in a two layer film wrap, where both layers have a carrier region positioned at least on one side of each layer, the total number of positioning combinations in the two layer stack is 25 (5 x 5) leading to different modes of active release. Yet, in a two layer film wrap which has a carrier region positioned in at least only one film layer and at least only on one side of the layer, the total number of positioning combinations in the two layer stack is 45 (5 x 9) leading to different modes of active release.

Although certain embodiments have been described herein, with reference to various lengths of film, it will be understood by those of skill in the art, that the length and width dimensions of the film vary with the particular device or application upon which the film is utilized, and can vary widely, from a film having a smallest dimension of about 0.05 cm (referring to either, length, width, or both) for smaller applications, to a film having a largest dimension of about 50 cm to 100 cm (referring to either length, width, or both) for larger applications. In a prospective example, a film for use with a 6 FR (2 mm in diameter) vascular catheter has a dimension ranging from about 1cm x 1cm to about 3 cm x 3 cm, depending on the specifications of the individual film, e.g., a film that wraps, one, two, three times or more around the tissue will have a corresponding increased length, and the desired width can also vary. In another prospective example, a film for use with a voice prosthesis has a dimension ranging from about 0.4 cm x 2.5 cm to about 2 cm x 10 cm, depending on the specifications of the individual film, e.g., a film that wraps, one, two, three times or more around the device will have a corresponding increased length, and the desired width can also vary. Yet, in another prospective example, a film for use with endotracheal tube, 9 mm in diameter, has a dimension ranging from about 1cm x 4 cm to about 3 cm x 12. cm, depending on the specifications of the individual film, e.g., a film that wraps, one, two, three times or more around the device will have a corresponding increased length, and the desired width can also vary. However, other film lengths and widths are envisioned to be within the scope of the invention and are not limited by the above-described examples, as will be understood by those of skill in the art, with reference to this disclosure.

### EXAMPLES

### Example 1. Preparation of pharmaceutical composition for active carrier member comprising antimicrobial peptide (AMP).

Polymer stock solution was prepared by dissolving 15 grams of a medical grade polyurethane (PU) in 85 grams of organic solvent. AMP stock solution was prepared by mixing 1 g of AMP, 1 g of a pharmaceutically acceptable pH regulator and 1 g of a pharmaceutically acceptable surfactant in 15 g of an alcohol solvent to obtain a homogeneous solution. The polymer stock (20 g) was mixed with the AMP stock (10 g) to obtain a homogeneous liquid referred to as the AMP-polymer stock.

### Example 2. Preparation of pharmaceutical composition for the active carrier member comprising anesthetic (lidocaine).

Polymer stock solution was prepared by dissolving 15 grams of a medical grade polyurethane (PU) in 85 grams organic solvent. Lidocaine stock solution was prepared by mixing 3 g of lidocaine with 15 g of an alcohol solvent to obtain a homogeneous solution. The polymer stock (20 g) was mixed with the lidocaine stock (10 g) to obtain a homogeneous liquid referred to as the anesthetic-polymer stock.

### Example 3. Preparation of composition for the blank region (member).

Polymer solution was prepared by dissolving 15 grams of a medical grade polyurethane (PU) in 85 grams of organic solvent.

The obtained polymer solution was cast on a polyester release liner and dried to form a flexible, non-tacky sheet that does not contain actives or adhesive.

### Example 4. Preparation of adhesive wrap with antimicrobial carrier member using transfer adhesive.

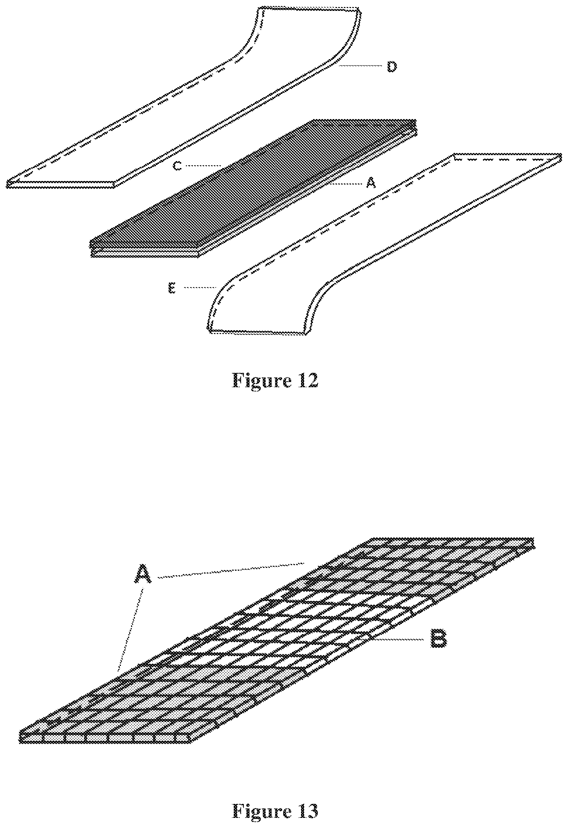

Referring now to Figure 12, a top perspective view of an exemplary adhesive film with an antimicrobial carrier member is shown, according to one embodiment, where **C-** AMP carrier. **A-** adhesive. **D-** removable polyester release liner, and **E-** removable paper carrier. In one embodiment, the adhesive film shown in Figure 12 was prepared by first, casting the AMP-polymer stock described in Example 1 on a siliconized polyester release liner and drying to form a sheet 4 inches wide and 5 inches long. A medical grade transfer adhesive 3M 1524A (3M Health Care) 6 inches wide on a siliconized paper carrier sheet was laminated to the AMP sheet with the AMP sheet being fully covered with the transfer adhesive (Figure 12). The AMP/adhesive strips, 20 mm in width and sandwiched between the polyester and the paper release liners, were cut to 15 mm length (short strip) and 40 mm length (long strip).

A medical grade silicone tube with outer diameter (OD) 4.9 mm (15.4 mm OD circumference) was cut into 5 cm long pieces. The polyester and the paper liners were removed from the AMP/adhesive strips, and the strips were applied on the silicone tube with the adhesive layer facing the surface of the silicone tube. The short (15 mm long) AMP/adhesive strip did not have sufficient length to form a loop around the silicone tube OD and did not overlap to adhere to itself. The long (40 mm long) AMP/adhesive strip (wrap) after being applied formed a loop around the silicone tube OD with the overlapping self-adhering area about 20 mm by 2.4 mm.

The samples of silicone tubes with the short (not overlapping) and the long (overlapping) AMP/adhesive strips were immersed in 0.9% saline solutions in closed vials and placed on a shaker inside an incubator chamber at 37 °C. The sample with the short (not overlapping) AMP/adhesive strip started detaching from the silicone tube after one day of soaking in saline and completely detached after 3 days, whereas the sample with the long (overlapping) AMP/adhesive strip remained in place on the silicone tube for more than 1 week without signs of detachment or sliding.

### Example 5. Preparation of adhesive wrap with antimicrobial carrier member using adhesive solution.

The AMP-polymer stock described in Example 1 was cast on a siliconized polyester release liner and dried to form an AMP sheet 10,16 cm (4 inches) wide and 12,7 cm (5 inches) long. A medical grade DURO-TAK 387-2510 J 87-2510 (Henkel Corporation) in ethyl acetate/hexane solvent was dispensed on top of the AMP sheet with a syringe and spread using a casting knife, the AMP sheet being fully covered with the adhesive (Figure 12). After drying, the adhesive surface was covered with siliconized paper carrier. AMP/adhesive strips 20 mm in width sandwiched between protective polyester and paper liners were cut to 50 mm length.

A medical grade polyvinyl chloride (PVC) tube with outer diameter (OD) 9.5 mm (29.8 mm OD circumference) was cut into 5 cm long pieces. The polyester and paper release liners were removed from the AMP/adhesive strip, and the strip was applied on the PVC tube with the adhesive layer facing the surface of the tube. The 50 mm long AMP/adhesive strip (wrap) after being applied formed a loop around the PVC tube OD with the overlapping self-adhering area about 20 mm by 20 mm.

### Example 6. Preparation of adhesive member on a mesh.

Referring now to Figure 13, a top perspective view of an exemplary adhesive member on a mesh is shown, where A- Mesh regions impregnated with adhesive, and **B-** mesh region not impregnated. The film shown in Figure 13 was prepared by first laying a polyethylene mesh strip a polyester release liner. About 1/3 of the mesh strip length from both ends were covered with DOW CORNING^{®} BIO-PSA 7-4601 silicone adhesive solution in heptane by dispensing the adhesive solution using a syringe, and the remaining about 1/3 of the strip in the middle was not impregnated with the adhesive (*see,* Figure 13). After drying, the adhesive member was covered with a second protective polyester release liner.

### Example 7. Preparation of self-adhesive wrap on a mesh with two active carrier regions.

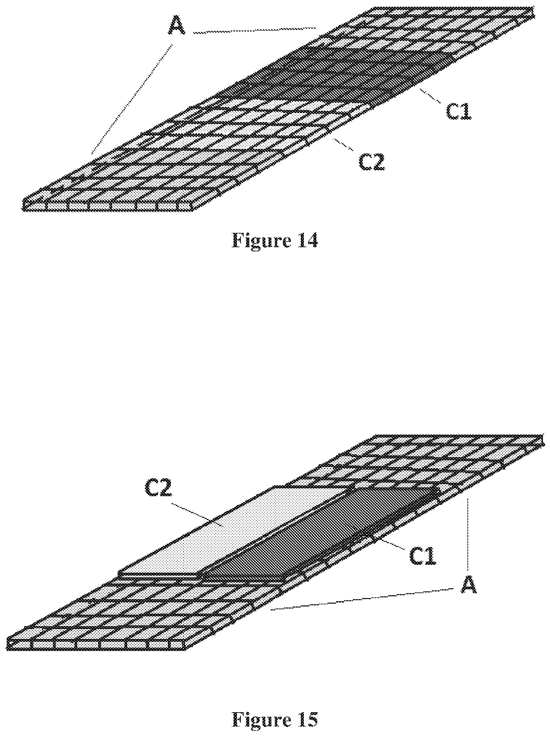

Referring now to Figure 14, a top perspective view of an exemplary self-adhesive film (wrap) with two active carrier regions is shown, where **C1**- mesh region impregnated with antimicrobial, **C2-** mesh region impregnated with anesthetic, and **A-** Mesh regions impregnated with adhesive. The wrap shown in Figure 14 was prepared by first, laying a nylon mesh strip on a polyester release liner. Then, ¼ of the mesh length from the first end and ¼ of the mesh length from the second end were covered and impregnated with a medical grade DURO-TAK 387-2510 / 87-2510 (Henkel Corporation) adhesive in ethyl acetate/hexane solvent by dispensing from a syringe and drying (regions A in Figure 14). Then the ¼ of the mesh length remaining uncovered by the adhesive was impregnated with antimicrobial liquid composition described in Example 1 and dried (region C1 in Figure 14.). Then the remaining uncovered (not impregnated) ¼ of the mesh length was impregnated with anesthetic liquid composition described in Example 2 and dried (region C2 in Figure 14.). The obtained self-adhesive wrap with 2 active carrier regions was covered with second protective polyester release liner.

### Example 8. Preparation of self-adhesive wrap on a mesh with two active carrier regions (members).

Referring now to Figure 15, a top perspective view of an exemplary self-adhesive film (wrap) with two active carrier regions is shown, where **C1**- antimicrobial carrier region, **C2-**anesthetic carrier region, and **A-** mesh impregnated with adhesive. The wrap shown in Figure 14 was prepared by first, laying a polyethylene mesh strip 16 cm long and 8 cm wide on a polyester release liner. Then, the mesh strip was fully covered and impregnated with a medical grade DURO-TAK 387-2510 / 87-2510 (Henkel Corporation) adhesive solution in ethyl acetate/hexane solvent by dispensing from a syringe and drying (member A in Figure 15).

The AMP-polymer stock described in Example 1 was cast on polyester release liner and dried to form a sheet. A 8 cm long and 4 cm wide strip was cut to form the antimicrobial carrier region member and laminated along one edge in the middle of the 16 cm by 8 cm adhesive member to form the antimicrobial carrier region (C1 in Figure 15.).

The anesthetic-polymer stock described in Example 2 was cast on polyester release liner and dried to form a sheet. A 8 cm long and 4 cm wide strip was cut to form the anesthetic carrier region member and laminated along one edge in the middle of the 16 cm by 8 cm adhesive member (A in Figure 15.), adjacent to the antimicrobial carrier region (C1 in Figure 15.) to form the anesthetic carrier region (C2 in Figure 15.).

The obtained self-adhesive wrap with two active carrier regions was covered with second protective polyester release liner.

### Example 9. Preparation of self-adhesive wrap with two actives and four active carrier region members.

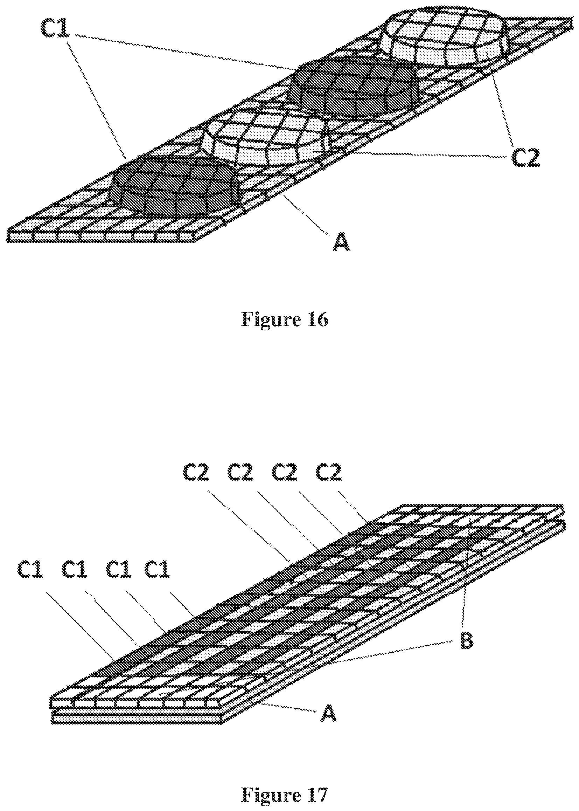

Referring now to Figure 16, a top perspective view of an exemplary self-adhesive film (wrap) with two actives and four active carrier regions is shown, where **C1**- antimicrobial carrier on mesh, **C2-** anesthetic carrier on mesh, and **A-** adhesive on mesh. The wrap shown in Figure 16 was prepared by first, laying a nylon mesh strip 20 cm long and 4 cm wide on a polyester release liner. The mesh strip was fully covered and impregnated with a medical grade DURO.. TAK 387-2510 / 87-2510 (Henkel Corporation) adhesive solution in ethyl acetate/hexane solvent by dispensing from a syringe, spreading with a casting knife and drying (A in Figure 16).

Polyester mesh discs 1.25 inches (3.175 cm) in diameter were punched using a circular die. The two polyester mesh discs were impregnated by dipping in the antimicrobial composition described in Example 1 and drying to obtain antimicrobial carrier members (C1 in Figure 16).

Two other polyester mesh discs were impregnated by dipping in the anesthetic composition described in Example 2 and drying to obtain anesthetic carrier members (C2 in Figure 16).

Two antimicrobial carrier members and two anesthetic carrier members were laminated in alternating order along the length of the adhesive member with the edge of first and the forth active carrier discs positioned approximately 2 cm from the edges of adhesive member and with spacing between discs of 2-3 mm (Figure 16).

### Example 10. Preparation of self-adhesive wrap with two actives and eight active carrier regions.

Referring now to Figure 17, a top perspective view of an exemplary self-adhesive film (wrap) with two actives and eight active carrier regions is shown, where **C1-** antimicrobial carrier on mesh, **C2-** anesthetic carrier on mesh, and **A-** adhesive on mesh. The wrap shown in Figure 17 was prepared by first, laminating a polyester mesh sheet 10,16 cm (4 inches) wide and 12,7 cm (5 inches) long (member B in Figure 17.) to a medical grade transfer adhesive 3M 1524A (3M Health Care) 10,16 cm (4 inches) wide and 12,7 cm (5 inches) long (member A in Figure 17.) on a siliconized paper carrier sheet. The polyester mesh/3M 1524A adhesive laminate on the paper carrier sheet were cut to 16 cm long and 8 cm wide strips and positioned on a glass slide with the polyester mesh member facing up.

The liquid antimicrobial composition described in Example 1 was dispensed from a syringe on the polyester mesh side of the laminate starting 2 cm from the mesh edge (proximal edge) to form a pattern of four stripes about 12 cm long and 1 cm wide with blank spacing between stripes about 1 cm, and stripes ending at about 2 cm from the second mesh edge (regions C1 in Figure 17). After drying the pattern of antimicrobial regions of four stripes impregnating the mesh was obtained on the mesh/adhesive laminate.

Blank spaces about 1cm wide and 12 cm long between the antimicrobial regions on the polyester mesh side of the laminate were covered (impregnated) with liquid anesthetic composition described in Example 2 and dried to form a pattern of four stripes comprising anesthetic carrier regions (regions C2 in Figure 17). After drying the pattern of anesthetic carrier regions of four stripes alternating with the four stripes of the antimicrobial carrier regions was obtained on the mesh/adhesive laminate as shown in the Figure 17.

### Example 11. Preparation of self-adhesive wraps with overlapping regions to control mode of actives release and dosing.

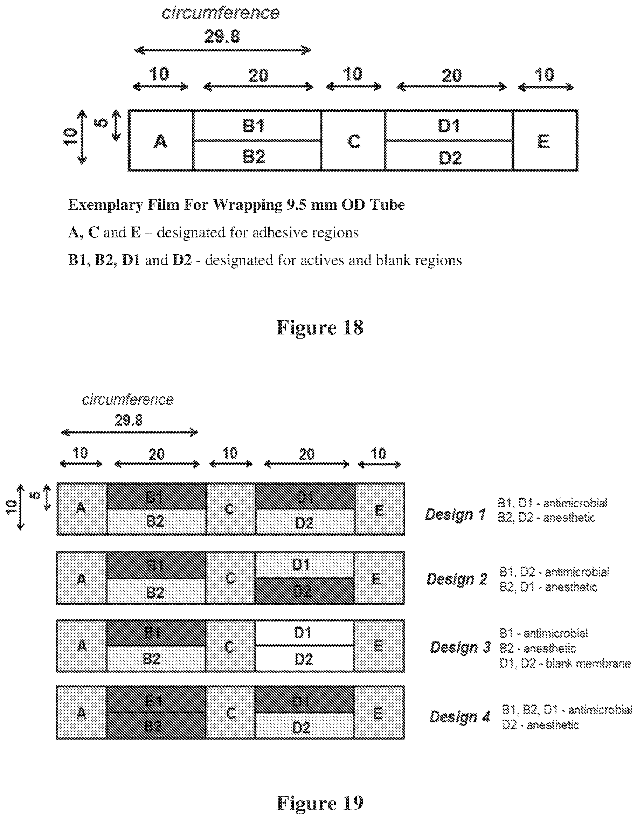

Referring now to Figure 18, a schematic top view of a film (wrap) showing regions pattern on the film suitable for wrapping a medical tube with a 9.5 mm OD, where **A, C** and **E** are designated for adhesive regions, **B1, B2, D1** and **D2** are designated for actives and blank regions. The film shown in Figure 18 is a schematic for a 70 mm long and 10 mm wide strip suitable for wrapping a medical grade tube with an outer diameter (OD) of 9.5 mm and an OD circumference of 29.8 mm. A pattern of the following regions suitable for creating adhesive, active carrier and blank regions in the wrap are shown in Figure 18 from left to right along the wrap strip: region **A** -10 mm by 10 mm, regions **B1** and **B2** - 20 mm by 5 mm, region **C** - 10 mm by 10 mm, regions **D1** and **D2** - 20 mm by 5 mm, and region **E** - 10 mm by 10 mm. When applying the wrap on the tube with 9.5 mm OD by first attaching the **A** region with the wrap axis perpendicular to the tube axis and then winding the wrap around the tube OD, the region C will overlap the region **A, D1** will overlap **B1, D2** will overlap **B2,** and region E will overlap region **C.**

Referring now to Figure 19, a schematic top view for film wrap designs (Design 1, Design 2, Design 3, and Design 4) is shown, where: A, C and E - adhesive regions in all four designs. In Design 1, B1 and D1 are antimicrobial regions and B2 and D2 are anesthetic regions. In Design 2, B1 and D2 are antimicrobial regions and B2 and D1 are anesthetic regions. In Design 3, B1 is an antimicrobial region and B2 is an anesthetic region, D1 and D2 are blank regions. In Design 4, B1, B2, and D1 are antimicrobial regions and P2. is an anesthetic region.

The four wrap (film) designs shown in Figure 19 were prepared on a polyester mesh strip 70 mm long by 10 mm wide as follows:
Regions **A, C** and **E** (Figure 13 and Figure 14) on the mesh strip were covered and impregnated with medical grade DURO-TAK 387-2510 / 87-2510 (Henkel Corporation) in ethyl acetate/hexane solvent by dispensing the adhesive solution using a syringe and following drying.

Antimicrobial liquid composition described in the Example 1, anesthetic liquid composition described in the Example 2 and polymer solution described in the Example 3 were used to create respective active and blank regions by dispensing corresponding solutions in designated regions on the mesh strip and following drying.

The impact of distribution of active regions in the wrap on mode of actives release and dosing is shown in the Table 1.

| **Table 1**. Impact of distribution of active regions in the wrap on mode of actives release and dosing. | | | | | |
|---|---|---|---|---|---|
| ***Design 1*** | **region** | **active agent** | **active dose** | **mode of release** | **total dose of actives** |
| Stack 1 | B1 | antimicrobial | 1X dose | Mode 1 + Mode 2 antimicrobial | 2X dose antimicrobial + 2Y dose anesthetic |
| | D1 | antimicrobial | 1X dose | | |
| Stack 2 | B2 | anesthetic | 1Y dose | Mode 1 + Mode 2 anesthetic | |
| | D2 | anesthetic | 1Y dose | | |
| | | | | | |

| ***Design 2*** | **region** | **active agent** | **active dose** | **mode of release** | **total dose of actives** |
|---|---|---|---|---|---|
| Stack 1 | B1 | antimicrobial | 1X dose | Mode 3 antimicrobial | 2X dose antimicrobial + 2Y dose anesthetic |
| | D1 | anesthetic | 1Y dose | Mode 1 anesthetic | |
| Stack 2 | B2 | anesthetic | 1Y dose | Mode 3 anesthetic | |
| | D2 | antimicrobial | 1X dose | Mode 1 antimicrobial | |
| | | | | | |

| ***Design 3*** | **region** | **active agent** | **active dose** | **mode of release** | **total dose of actives** |
|---|---|---|---|---|---|
| Stack 1 | B1 | antimicrobial | 1X dose | Mode 4 antimicrobial | 1X close antimicrobial + 1Y dose anesthetic |
| | D1 | blank | none | membrane for B 1 | |
| Stack 2 | B2 | anesthetic | 1Y dose | Mode 4 anesthetic | |
| | D2 | blank | none | membrane for B2 | |
| | | | | | |

| ***Design 4*** | **region** | **active agent** | **active dose** | **mode of release** | **total dose of actives** |
|---|---|---|---|---|---|
| Stack 1 | B1 | antimicrobial | 1X | Mode 1 + Mode 2 antimicrobial | 3 X dose antimicrobial + 1Y close anesthetic |
| | D1 | antimicrobial | 1X | | |
| Stack 2 | B2 | antimicrobial | 1X | Mode 3 antimicrobial | |
| | D2 | anesthetic | 1Y | Mode 1 anesthetic | |

### Example 12. Preparation of self-adhesive films with antimicrobial peptide ASP1 and lidocaine anesthetic suitable for wrapping endotracheal tubes.

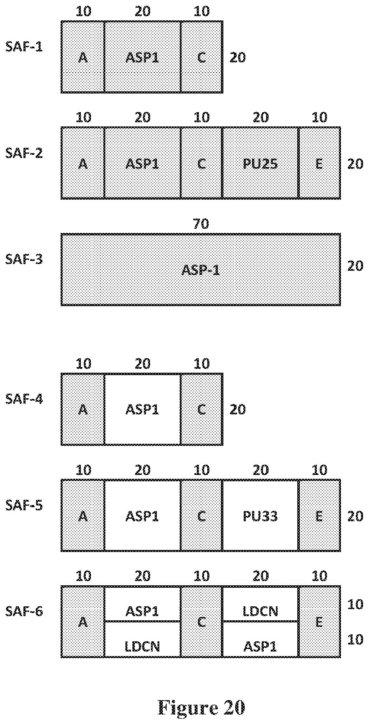

Referring now to Figure 20, a schematic top view of various self-adhesive films designed for wrapping endotracheal tubes (9.5 mm OD) with the top facing device surface is shown, where: Grey shaded areas are regions covered with adhesive on top; ASP1 are regions with antimicrobial peptide; PU25 are blank regions with polyurethane PU25, PU33 are blank regions with polyurethane PU33, LDCN - are regions with lidocaine anesthetic. Dimensions are shown in mm.

Figure 20 is an exemplary depiction of six designs of self-adhesive films that were prepared for wrapping Mallinckrodt Hi-Lo Oral/Nasal Tracheal tube, cuffed, 7.0 mm ID, 9.5 mm OD (REF # 86111). The obtained self-adhesive films 40 mm x 20 mm in size (designs SAF-1 and SAF-4) were suitable for wrapping the endotracheal tubes once, for example above the inflatable cuff, whereas films 70 mm × a 20 mm in size (designs SAF-2, SAF-3, SAF-5 and SAF--6) are suitable for wrapping the endotracheal tubes twice.

The following components were used to prepare the self-adhesive films:
medical grade polyethylene (PE) mesh (Delnet X540-S, DelStar Technologies);
medical grade adhesive (1504XL, 3M Corporation);
proprietary medical grade hydrophilic polyurethane PU2.5;
proprietary medical grade hydrophilic polyurethane PU33;
proprietary medical grade hydrophilic polyurethane PU77;

ASP1 antimicrobial peptide (AMP) with amino acid sequence RRWVRRVRRWVRRV VRVVRRWVRR and purity> 95%;

ASP1 films were prepared by casting and drying liquid formulation from an organic solvent comprising ASP1 antimicrobial peptide, proprietary medical grade hydrophilic polyurethane PU 77 and pharmaceutical grade excipients.

LDCN films were prepared by casting and drying liquid formulation from an organic solvent comprising lidocaine and proprietary medical grade hydrophilic polyurethane PU33; PU33 films were cast from an organic solvent and dried;

PU25 films were cast from an organic solvent and dried.

### Preparation of SAF-1 and SAF-2 wraps.

Templates for SAF-1 and SAF-2 designs were printed and positioned on a 15, 24cm X 25, 4cm (6''X 10") glass slab. Clear polyester (PET) release liner (PN 1022-0600 0-100, Fralock Corp.) 12,7 cm × 20, 32 cm (5" × 8") in size was positioned on top of templates with the siliconized surface of the liner facing up and fixed with tape outside the templates areas. Delnet (PE) mesh 12,7 cm × 20, 32 cm (5"× 8") in size was then positioned on top of the release liner and fixed with tape outside the templates areas. ASP1 films about 80 microns thick on PET release liner carrier were cut to required sizes, wetted with an organic solvent and laminated to Delnet PE meshes to create ASP1 regions as shown in the Figure 20. On SAF-2 templates PU25 films about 90 microns thick were cut to required sizes, wetted with an organic solvent on a release liner carrier and laminated to Delnet PE meshes to create blank regions marked PU25 in the Figure 20. Release liners were removed from ASP1 films and PU25 films, the regions were allowed to dry from the solvent in a convection oven. The 3M adhesive film sandwiched between two release liners was cut to sizes 40 mm × 20 mm for SAF-1 film and 70 mm x 20 mm for SAF-2, then release liner from one side was removed and adhesive was laminated on the PE mesh/ASP1 constructs over the whole film areas: A, ASP1 and C for SAF-1, and areas A, ASP1, C, PLT25 and E for the SAF-2 as marked with grey color in the Figure 20. Excess of PE Delnet mesh was cut away from the constructs along borders of the templates to obtain final self-adhesive films covered with release liners.

The obtained SAF-1 and SAF-2 wraps were applied on the endotracheal tubes (ETT, 9.5 mm OD) above the cuffs as follows: release liners were removed and the A areas were first applied with adhesive facing ETT surface followed by wrapping the films around ETT tube once for the SAF-1 and twice for the SAF-2.

### Preparation of SAF-3 wraps.

Clear polyester (PET) release liner (PN 1022-0600 0-100, Fralock Corp.) 12,7 cm × 20,32 cm (5"× 8") in size was positioned on a 15,24 cm X 25,4cm (6"× 10") glass slab with siliconized side facing up and fixed with a tape. ASP1 film about 80 microns thick was cut to 70 mm x 20 mm size and placed on the glass slide with the release liner. The ASP1 film then was covered with about 30 mm × 80 mm 3M adhesive strip on a release liner carrier, excess of 3M adhesive was then cut away along the borders of the ASP1 film to obtain final SAF-3 construct.

The obtained SAF-3 film was applied on the endotracheal tube (ETT, 9.5 mm OD) above the inflatable cuff as follows: release liner was removed, and one end of the film was first laminated with the adhesive facing ETT surface followed by wrapping the film around ETT tube twice.

### Preparation of SAF-4, SAF-5 and SAF-6 wraps.

Templates for SAF-4, SAF-5 and SAF-6 designs were printed and positioned on a 15,24cm X 25,4cm (6"× 10") glass slab. Clear polyester (PET) release liner (PN 1022-0600 0-100, Fralock Corp.) 12,7 cm X 20,32 cm (5"× 8") in size was positioned on top of templates with the siliconized surface of the liner facing up and fixed with tape outside the templates areas. Delnet (PE) mesh 12,7 cm X 20,32 cm (5"× 8") in size was then positioned on top of the release liner and fixed with tape outside the templates areas. ASP1 films about 80 microns thick on PET release liner carrier were cut to required sizes, wetted with an organic solvent and laminated to Delnet PE meshes to create ASP1 regions as shown in the Figure 20. PU33 films about 110 microns thick were cut to required sizes, wetted with an organic solvent and laminated to Delnet PE meshes to create blank regions as shown in the Figure 20. LDCN films about 90 microns thick were cut to required sizes, wetted with an organic solvent and laminated to Delnet PE meshes to create lidocaine regions as shown in the Figure 20. Release liners were removed from ASP1 regions, PU33 regions and LDCN regions, the regions were allowed to dry from the solvent in a convection oven. The 3M adhesive film sandwiched between two release liners was cut to sizes, release liner from one side was removed and adhesive was laminated on areas A, C and E as shown in Figure 20 in grey shaded areas.

The obtained wraps were applied on the ETTs (9.5 mm OD) above the inflatable cuffs as follows: release liners were removed from adhesive areas A, C and E, then A areas were first applied with adhesive facing ETT surface followed by wrapping the films around ETT tube once for the SAF-4 and twice for the SAF-5 and SAF-6 with C areas overlapping A areas and E areas overlapping C areas.

Release of actives from ETT wraps was studied over 4 days. Wrapped ETT segments were cut from ETTs and placed in tubes containing 5 mL of 0.9% saline buffered with acetate at pH 6.0. The samples were placed for extraction in triplicates under mild shaking inside incubator at 37 °C at time points 3 hours, 1 day, 2, 3 and 4 days the 5 mL extracts were removed for actives assay and replaced with fresh 5 mL saline buffer to continue release experiments. The extracts were analyzed for ASP1 peptide and lidocaine content using RP-HPLC (Waters Corp. Alliance system with Waters e2695 separation module and Waters 2998 PDA detector).

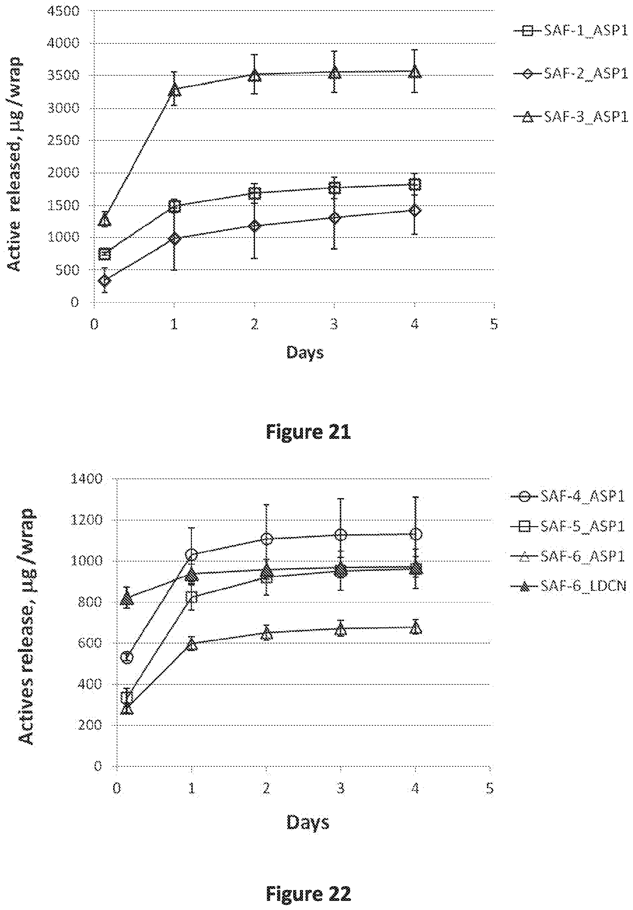

Referring now to Figure 21, a plot of ASP1 antimicrobial peptide release from ETT wraps SAF-1, SAF-2 and SAF-3 (n=3), shown in Figure 20, is shown. Referring now to Figure 22, a plot of actives released from ETT wraps SAF-4, SAF-5 and SAF-6 (n=3), shown in Figure 20, is shown, where ASP1 is antimicrobial peptide, and LDCN is lidocaine.

In all cases wraps remained firmly in place without signs of detachment or de-lamination in the course of experiments. The release of ASP1 antimicrobial peptide from the obtained ETT wraps is shown in Figure 21 and Figure 22. Lidocaine simultaneously released with ASP1 from the SAF-6 wrap, as shown in Figure 22.

As is understood by those of skill in the art, the self-adhesive wraps for endotracheal tubes presented herein are for the purpose of describing particular embodiments only, and are not intended to be limiting to particular materials, compositions, adhesives, bioactive agents, devices, film shapes, film structures and the like.

### Example 13. Preparation of self-adhesive films with antimicrobial peptide ASP1 and for wrapping wound drain tubes.

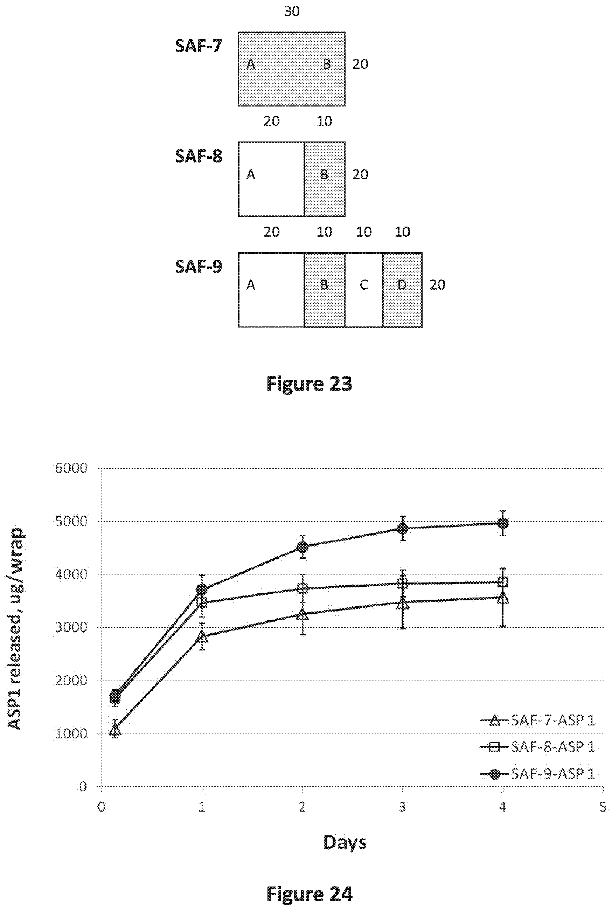

Referring now to Figure 23, a schematic top view of various self-adhesive films designed for wrapping 19Fr wound drain tubes with the top facing device surface is shown. The films are made of polyurethane PU25 loaded with ASP1 antimicrobial peptide. Grey shaded areas depict film regions covered with adhesive on top. Figure 23 is an exemplary depiction of three designs of self-adhesive films comprising antimicrobial peptide ASP1 that were prepared for wrapping 19Fr (6.3 mm OD) silicone wound drain tubes (Medline Industries, Inc.). The obtained self-adhesive films 30 mm x 20 mm in size (designs SAF-7 and SAF-8) are suitable for wrapping the 19Fr wound drain tubes once, for example at tube spot corresponding to body entry site, whereas films 50 mm x 20 mm in size (design SAF-9) are suitable for wrapping the 19Fr wound drain tubes twice.

The following components were used to prepare the self-adhesive films shown in Figure 23:
3M^{™} Silicone Adhesive Transfer Tape 91022. (3M Corporation);
proprietary medical grade hydrophilic polyurethane PU25;
ASP1 antimicrobial peptide (AMP) with amino acid sequence RRWVRRVRRWVRRV VRVVRRWVRR and purity> 95%;
ASP1 films were prepared by casting and drying liquid formulation from an organic solvent, dry films comprising 8.6 wt%ASP1 antimicrobial peptide mixed with pharmaceutical excipients in proprietary medical grade hydrophilic polyurethane PU 25.

### Preparation of SAF-7, SAF-8 and SAF-9 wraps.

Clear polyester (PET) release liner (PN 1022-0600 0-100, Fralock Corp.) 12,7 cm X 20,32 cm (5"× 8") size was positioned on a 15,24 cm × 25,4cm (6"× 10") glass slab with siliconized side facing up and fixed with a tape. ASP1 films about 100 microns thick on a support PET release liner were cut to 30 mm x 20 mm strips (for SAF-7 and SAF-8 designs) or 50 mm x 20 mm strips (for SAF-9) and placed on the release liner mounted on the glass. To obtain SAF-7 the whole 30 mm x 20 mm ASP1 film then was covered with 30 mm × 20 mm 3M Silicone Adhesive Transfer Tape. To obtain SAF-8 the 3M adhesive transfer tape 10 mm x 20 mm in size was laminated to the B-end of the film as shown in grey shaded area in Figure 23. To obtain SAF-9 two 3M adhesive transfer tapes 10 mm x 20 mm in size were laminated on the D-end and the B -middle regions as shown in grey shaded areas in Fig 23. The A-ends of the self-adhesive films that either had adhesive (SAF-7) or did not have adhesive (SAF-8 and SAF-9) were applied first on the 19Fr wound drain tubes (Figure 23) followed by wrapping once (SAF-7 and SAF-8) or twice (SAF-9) with the adhesive regions facing towards the surface of the drain tubes.

The release of antimicrobial ASP-1 peptide from the wraps SAF-7, SAF-8, and SAF-9 was studied over 4 days. Wrapped silicone tubing segments were placed in tubes containing 5 mL of 0.9% saline buffered with acetate at pH 6.0. The samples were placed for extraction in triplicates under mild shaking inside incubator at 37 °C at time points 3 hours, 1 day, 2, 3 and 4 days the 5 mL extracts were removed for actives assay and replaced with fresh 5 mL saline buffer to continue release experiments. The extracts were analyzed for ASP1 peptide content using RP-HPLC (Waters Corp. Alliance system with Waters e2695 separation module and Waters 2998 PDA detector).

Referring now to Figure24, a plot of the release of ASP1 antimicrobial peptide from the wound drain tube wraps SAF-7, SAF-8 and SAF-9 (n=3), shown in Figure 23, is shown.

In all cases wraps remained firmly in place without signs of detachment or de-lamination in the course of experiments. The release of ASP1 antimicrobial peptide from the obtained wound drain tube wraps is shown in Figure 24 over four days, with samples taken at 3 hours, 1, 2, 3, and 4 days.

## Claims

1. A film for self-adhering to a surface of a three-dimensional object and delivery of one or more bioactive agents within a body, the film comprising:
a film comprising one or more layers, where the film and each film layer has a first side and an opposite second side;
a width, having a width dimension;
a first end portion and a second end portion, the first and second end portion positioned at opposite ends of the length of the film;
a length, having a length dimension which is of sufficient length such that the film wraps at least once around an outer surface of the three-dimensional object with the first and second end portions at least partially overlapping;
a plurality of component regions positioned on, or within, or partially with, or a combination thereof, the first and second sides of the film layers, the plurality of component regions selected from the group consist of:
one or more a carrier regions, each carrier region, individually, comprising one or more bioactive agents, the one or more bioactive agents being the same or different, wherein the bioactive agent is releasable from the one or more carrier regions when the film is wrapped around the three-dimensional object when the film is positioned within a body;
one or more adhesive regions, each adhesive region extending at least a portion of the width of the film; and
one or more blank regions which does not contain adhesive or bioactive agent,
wherein:
the first side of the film comprises at least a first component region and a second component region, which are the same or different, wherein at least one of the first and second component regions is a carrier region, and
the second side of the film comprises (i) at least a first adhesive region positioned on the first or the second end portion of the film, the first adhesive region being capable of self-adhering when the film engages at the opposite end portion of the film when the film is wrapped at least once around said three-dimensional object; and (ii) one or more blank regions or one or more carrier regions.

2. The film according to claim 1, wherein the film comprises one or more blank regions positioned on a layer which does not contain adhesive or a bioactive agent..

3. The film according to claim 1 or 2 further comprising a second adhesive region, the second adhesive region being positioned in between the end portions of the film.

4. The film according to any of the preceding claims, wherein at least one carrier region is a layer positioned on the second side of the film and defines least in part an outermost layer of the film; or
wherein at least one carrier region is a layer positioned on the first side of the film and defines least in part an innermost layer of the film.

5. The film according to any of the preceding claims wherein the one or more carrier regions comprises a first carrier region and a second carrier region, the first carrier region comprising a first pharmaceutical composition having a first bioactive agent, and the second carrier region comprising a second pharmaceutical composition having a second bioactive agent;
wherein, optionally, the first and second carrier regions are separated by a blank region, each blank region comprising a film layer which does not contain adhesive or a bioactive agent.

6. The film according to any of the preceding claims, wherein the one or more a carrier regions comprises at least one carrier region that comprises two or more different bioactive agents positioned on the same carrier region.

7. The film according to claim 1 wherein the film comprises a material selected from the group consisting of a flexible polymeric material,
a porous flexible film, a mesh, a non-woven or woven fabric, a sponge, and a foam.

8. The film according to claim 1 wherein the self-adhesive film comprises a length dimension of sufficient length to wrap around the surface of the three-dimensional object at least twice to form at least two overlapping layers, where the end portion of the film, comprising the first adhesive region, self-adheres to the underlying film layer to form an overlapping multilayer film structure.

9. The film according to claim 1 or 8, wherein the bioactive agent is released from the carrier region of the film when the film is wrapped around the three-dimensional object and positioned within a body and the bioactive agent is released from the film into the body, or
wherein the bioactive agent is released from the carrier region of the film when the film is wrapped around the three-dimensional object and positioned within a body and the bioactive agent is released from the film onto the surface of the three-dimensional object.

10. The film according to claim 8
wherein the one or more carrier regions comprises a plurality of carrier regions, each carrier region, individually, comprising one or more bioactive agents, the one or more bioactive agents being the same or different, and wherein each of the plurality of carrier regions, individually, is positioned on one or both of the first and second sides of the film; and
wherein the film further comprises a plurality of blank regions, each blank region comprising a layer which does not contain adhesive or a bioactive agent, the one or more blank regions being positioned on the first side or the second side of the film layer and in-between or adjacent to one of the plurality of carrier regions or the adhesive regions..

11. A film according to claim 10 wherein at least one of the plurality of carrier regions is positioned on the film such that it is directly atop another of the plurality of carrier regions when the film is wrapped around the three-dimensional object, or
wherein at least one of the plurality of blank regions is positioned on the film such that it is directly atop one of the plurality of carrier regions when the film is wrapped around the three-dimensional object and the blank region acts as a regulator for release of the bioactive agent, or
wherein at least one of the plurality of blank regions acts as a regulator for release of the bioactive agent when the film is wrapped around the three-dimensional object.

12. A device, tissue or anatomical structure having a self-adhesive film according to claim 1 for delivery of one or more bioactive agents within a body, the device, tissue or anatomical structure comprising:
a device, tissue or anatomical structure element having an outer surface and a substantially three-dimensional shape; and
a self-adhesive film according to claim 1.

13. The device, tissue or anatomical structure according to claim 12 wherein the device, tissue or anatomical structure is a device selected from the group consisting of catheters, vascular catheters, vascular grafts, arteriovenous grafts, urinary catheters, urinary stents, suprapubic catheters, tracheal tubes, esophagostomy tubes, trachea-esophageal voice prosthesis, peritoneal dialysis catheters, drainage tube/catheters, tympanostomy tubes, orthopedic implants, orthopedic fixation devices, pace makers, heart assist devices, neurostimulation devices, drug delivery devices, and feeding tubes.

14. The device, tissue or anatomical structure according to claim 12 wherein the device, tissue or anatomical structure is a tissue or anatomical structure selected from the group consisting of nerves, vessels, connective tissues including tendons, ligaments and bones, esophagus, intestine, colon, surgical anastomoses and fistulas.

## Patentansprüche

1. Folie zur Selbstklebung an einer Oberfläche eines dreidimensionalen Objekts und zur Abgabe eines oder mehrerer bioaktiver Wirkstoffe innerhalb eines Körpers, wobei die Folie Folgendes umfasst:
eine Folie, die eine oder mehrere Schichten umfasst, wobei die Folie und jede Folienschicht eine erste Seite und eine entgegengesetzte zweite Seite aufweisen;
eine Breite, die eine Breitenabmessung aufweist;
einen ersten Endabschnitt und einen zweiten Endabschnitt, wobei der erste und der zweite Endabschnitt an entgegengesetzten Enden der Länge der Folie positioniert sind;
eine Länge, die eine Längenabmessung aufweist, die ausreichend lang ist, sodass die Folie sich mindestens einmal um eine Außenfläche des dreidimensionalen Objekts wickelt, wobei der erste und der zweite Endabschnitt mindestens teilweise überlappen;
eine Vielzahl von Komponentenbereichen, die auf, oder innerhalb oder teilweise mit, oder eine Kombination davon, der ersten und der zweiten Seite der Folienschichten positioniert sind, wobei die Vielzahl von Komponentenbereichen aus der Gruppe ausgewählt ist, die aus Folgendem besteht:
einem oder mehreren Trägerbereichen, wobei jeder Trägerbereich einzeln einen oder mehrere bioaktive Wirkstoffe umfasst, wobei der eine oder die mehreren bioaktiven Wirkstoffe gleich oder unterschiedlich sind, wobei der bioaktive Wirkstoff von dem einen oder den mehreren Trägerbereichen freisetzbar ist, wenn die Folie um das dreidimensionale Objekt gewickelt wird, wenn die Folie innerhalb eines Körpers positioniert wird;
einem oder mehreren Klebebereichen, wobei sich jeder Klebebereich über mindestens einen Abschnitt der Breite der Folie erstreckt; und
einem oder mehreren leeren Bereichen, die weder Klebstoff noch bioaktiven Wirkstoff enthalten, wobei:
die erste Seite der Folie mindestens einen ersten Komponentenbereich und einen zweiten Komponentenbereich umfasst, die gleich oder unterschiedlich sind, wobei mindestens einer des ersten und des zweiten Komponentenbereichs ein Trägerbereich ist, und
die zweite Seite der Folie (i) mindestens einen ersten Klebebereich, der an dem ersten oder dem zweiten Endabschnitt der Folie positioniert ist, wobei der erste Klebebereich selbstklebend sein kann, wenn die Folie an dem entgegengesetzten Endabschnitt der Folie eingreift, wenn die Folie mindestens einmal um das dreidimensionale Objekt gewickelt wird; und (ii) einen oder mehrere leere Bereiche oder einen oder mehrere Trägerbereiche umfasst.

2. Folie nach Anspruch 1, wobei die Folie einen oder mehrere leere Bereiche umfasst, die auf einer Schicht positioniert sind, die weder Klebstoff noch bioaktiven Wirkstoff enthält.

3. Folie nach Anspruch 1 oder 2, die weiter einen zweiten Klebebereich umfasst, wobei der zweite Klebebereich zwischen den Endabschnitten der Folie positioniert ist.

4. Folie nach einem der vorstehenden Ansprüche, wobei mindestens ein Trägerbereich eine Schicht ist, die auf der zweiten Seite der Folie positioniert ist und mindestens zum Teil eine äußerste Schicht der Folie definiert; oder
wobei mindestens ein Trägerbereich eine Schicht ist, die auf der ersten Seite der Folie positioniert ist und mindestens zum Teil eine innerste Schicht der Folie definiert.

5. Folie nach einem der vorstehenden Ansprüche, wobei der eine oder die mehreren Trägerbereiche einen ersten Trägerbereich und einen zweiten Trägerbereich umfassen, wobei der erste Trägerbereich eine erste pharmazeutische Zusammensetzung umfasst, die einen ersten bioaktiven Wirkstoff aufweist, und der zweite Trägerbereich eine zweite pharmazeutische Zusammensetzung umfasst, die einen zweiten bioaktiven Wirkstoff aufweist;
wobei optional der erste und der zweite Trägerbereich durch einen leeren Bereich getrennt sind, wobei jeder leere Bereich eine Folienschicht umfasst, die weder Klebstoff noch bioaktiven Wirkstoff enthält.

6. Folie nach einem der vorstehenden Ansprüche, wobei der eine oder die mehreren Trägerbereiche mindestens einen Trägerbereich umfassen, der zwei oder mehr unterschiedliche bioaktive Wirkstoffe umfasst, die auf demselben Trägerbereich positioniert sind.

7. Folie nach Anspruch 1, wobei die Folie ein Material umfasst, das aus der Gruppe ausgewählt ist, die aus einem flexiblen Polymermaterial,
einer porösen flexiblen Folie, einem Netz, einem Vlies- oder Gewebestoff, einem Schwamm und einem Schaum besteht.

8. Folie nach Anspruch 1, wobei die selbstklebende Folie eine Längsabmessung umfasst, die ausreichend lang ist, um die Oberfläche des dreidimensionalen Objekts mindestens zweimal zu umwickeln, um mindestens zwei überlappende Schichten zu bilden, wobei der Endabschnitt der Folie, der den ersten Klebebereich umfasst, an der darunterliegenden Folienschicht selbst anklebt, um eine überlappende mehrschichtige Folienstruktur zu bilden.

9. Folie nach Anspruch 1 oder 8, wobei der bioaktive Wirkstoff aus dem Trägerbereich der Folie freigesetzt wird, wenn die Folie um das dreidimensionale Objekt gewickelt und innerhalb eines Körper positioniert wird und der bioaktive Wirkstoff aus der Folie in den Körper freigesetzt wird, oder
wobei der bioaktive Wirkstoff aus dem Trägerbereich der Folie freigesetzt wird, wenn die Folie um das dreidimensionale Objekt gewickelt und innerhalb eines Körper positioniert wird und der bioaktive Wirkstoff aus der Folie auf die Oberfläche des dreidimensionalen Objekts freigesetzt wird.

10. Folie nach Anspruch 8,
wobei der eine oder die mehreren Trägerbereiche eine Vielzahl von Trägerbereichen umfassen, wobei jeder Trägerbereich einzeln einen oder mehrere bioaktive Wirkstoffe umfasst, wobei der eine oder die mehreren bioaktiven Wirkstoffe gleich oder unterschiedlich sind, und wobei jeder der Vielzahl von Trägerbereichen einzeln auf einer oder beiden der ersten und zweiten Seiten der Folie positioniert ist; und
wobei die Folie eine Vielzahl von leeren Bereichen umfasst, wobei jeder leere Bereich eine Schicht umfasst, die weder Klebstoff noch einen bioaktiven Wirkstoff enthält, wobei die ein oder mehreren leeren Bereiche auf der ersten Seite oder der zweiten Seite der Folienschicht und zwischen oder neben einem der Vielzahl von Trägerbereichen oder den Klebebereichen positioniert sind.

11. Folie nach Anspruch 10, wobei mindestens einer der Vielzahl von Trägerbereichen so auf der Folie positioniert ist, dass er direkt auf einem anderen der Vielzahl von Trägerbereichen liegt, wenn die Folie um das dreidimensionale Objekt gewickelt ist, oder
wobei mindestens einer der Vielzahl von leeren Bereichen so auf der Folie positioniert ist, dass er direkt auf einem der Vielzahl von Trägerbereichen liegt, wenn die Folie um das dreidimensionale Objekt gewickelt ist, und der leere Bereich als Regulator für eine Freisetzung des bioaktiven Wirkstoffs fungiert, oder
wobei mindestens einer der Vielzahl von leeren Bereichen als Regulator für die Freisetzung des bioaktiven Wirkstoffs fungiert, wenn die Folie um das dreidimensionale Objekt gewickelt ist.

12. Vorrichtung, Gewebe oder anatomische Struktur, die/das eine selbstklebende Folie nach Anspruch 1 zur Abgabe eines oder mehrerer bioaktiver Wirkstoffe innerhalb eines Körpers aufweist, wobei die Vorrichtung, das Gewebe oder die anatomische Struktur Folgendes umfasst:
eine Vorrichtung, ein Gewebe oder ein anatomisches Strukturelement, die/das eine Außenfläche und eine im Wesentlichen dreidimensionale Form aufweist; und
eine selbstklebende Folie nach Anspruch 1.

13. Vorrichtung, Gewebe oder anatomische Struktur nach Anspruch 12, wobei die Vorrichtung, das Gewebe oder die anatomische Struktur eine Vorrichtung ist, die aus der Gruppe ausgewählt ist, bestehend aus Kathetern, Gefäßkathetern, Gefäßtransplantaten, arteriovenösen Transplantaten, Harnkathetern, Harnleiterstents, suprapubischen Kathetern, Trachealtuben, Ösophagostomietuben, Tracheo-Ösophagus-Stimmprothesen, Peritonealdialysekathetern, Drainageschläuchen/-kathetern, Tympanotomieschläuchen, orthopädischen Implantaten, orthopädischen Fixiervorrichtungen, Herzschrittmachern, Herzunterstützungsvorrichtungen, Neurostimulationsvorrichtungen, Arzneimittelverabreichungsvorrichtungen und Ernährungssonden.

14. Vorrichtung, Gewebe oder anatomische Struktur nach Anspruch 12, wobei die Vorrichtung, das Gewebe oder die anatomische Struktur ein Gewebe oder eine anatomische Struktur ist, das/die der Gruppe ausgewählt ist, bestehend aus Nerven, Gefäßen, Bindegeweben, einschließlich Sehnen, Bändern und Knochen, Speiseröhre, Darm, Dickdarm, chirurgischen Anastomosen und Fisteln.

## Revendications

1. Film destiné à auto-adhérer à une surface d'un objet tridimensionnel et à administrer un ou plusieurs agents bioactifs à l'intérieur d'un corps, le film comprenant :
un film comprenant une ou plusieurs couches, dans lequel le film et chaque couche de film présentent une première face et une seconde face opposée ;
une largeur, présentant une dimension de largeur ;
une première partie d'extrémité et une seconde partie d'extrémité, les première et seconde parties d'extrémité étant positionnées à des extrémités opposées de la longueur du film ;
une longueur, présentant une dimension de longueur qui est d'une longueur suffisante pour que le film s'enroule au moins une fois autour d'une surface externe de l'objet tridimensionnel, les première et seconde parties d'extrémité se chevauchant au moins partiellement ;
une pluralité de régions de composants positionnées sur, ou à l'intérieur, ou partiellement avec, ou une combinaison de ceux-ci, les première et seconde faces des couches de film, la pluralité de régions de composants étant sélectionnées dans le groupe consistant en :
une ou plusieurs régions porteuses, chaque région porteuse, individuellement, comprenant un ou plusieurs agents bioactifs, les un ou plusieurs agents bioactifs étant identiques ou différents, dans lequel l'agent bioactif peut être libéré des une ou plusieurs régions porteuses lorsque le film enveloppe l'objet tridimensionnel lorsque le film est positionné à l'intérieur d'un corps ;
une ou plusieurs régions adhésives, chaque région adhésive s'étendant sur au moins une partie de la largeur du film ; et
une ou plusieurs régions vierges qui ne contiennent pas d'adhésif ni d'agent bioactif, dans lequel :
la première face du film comprend au moins une première région de composant et une seconde région de composant, qui sont identiques ou différentes, dans lequel au moins l'une des première et seconde régions de composants est une région porteuse, et
la seconde face du film comprend (i) au moins une première région adhésive positionnée sur la première ou la seconde partie d'extrémité du film, la première région adhésive étant apte à auto-adhérer lorsque le film entre en contact avec la partie d'extrémité opposée du film lorsque le film est enroulé au moins une fois autour dudit objet tridimensionnel ; et (ii) une ou plusieurs régions vierges ou une ou plusieurs régions porteuses.

2. Film selon la revendication 1, dans lequel le film comprend une ou plusieurs régions vierges positionnées sur une couche qui ne contient pas d'adhésif ni d'agent bioactif.

3. Film selon la revendication 1 ou 2, comprenant en outre une seconde région adhésive, la seconde région adhésive étant positionnée entre les parties d'extrémité du film.

4. Film selon l'une quelconque des revendications précédentes, dans lequel au moins une région porteuse est une couche positionnée sur la seconde face du film et définit au moins en partie une couche la plus externe du film ; ou
dans lequel au moins une région porteuse est une couche positionnée sur la première face du film et définit au moins en partie une couche la plus interne du film.

5. Film selon l'une quelconque des revendications précédentes, dans lequel les une ou plusieurs régions porteuses comprennent une première région porteuse et une seconde région porteuse, la première région porteuse comprenant une première composition pharmaceutique présentant un premier agent bioactif, et la seconde région porteuse comprenant une seconde composition pharmaceutique présentant un second agent bioactif ;
dans lequel, facultativement, les première et seconde régions porteuses sont séparées par une région vierge, chaque région vierge comprenant une couche de film qui ne contient pas d'adhésif ni d'agent bioactif.

6. Film selon l'une quelconque des revendications précédentes, dans lequel les une ou plusieurs régions porteuses comprennent au moins une région porteuse qui comprend deux agents bioactifs différents ou plus positionnés sur la même région porteuse.

7. Film selon la revendication 1, dans lequel le film comprend un matériau sélectionné dans le groupe consistant en un matériau polymère flexible,
un film flexible poreux, un maillage, un tissu non-tissé ou tissé, une éponge et une mousse.

8. Film selon la revendication 1, dans lequel le film auto-adhésif comprend une dimension de longueur de longueur suffisante pour s'enrouler autour de la surface de l'objet tridimensionnel au moins deux fois pour former au moins deux couches superposées, dans lequel la partie d'extrémité du film, comprenant la première région adhésive, auto-adhère à la couche de film sous-jacente pour former une structure de film multicouche superposée.

9. Film selon la revendication 1 ou 8, dans lequel l'agent bioactif est libéré de la région porteuse du film lorsque le film est enroulé autour de l'objet tridimensionnel et positionné à l'intérieur d'un corps et l'agent bioactif est libéré du film dans le corps, ou
dans lequel l'agent bioactif est libéré de la région porteuse du film lorsque le film est enroulé autour de l'objet tridimensionnel et positionné à l'intérieur d'un corps et l'agent bioactif est libéré du film sur la surface de l'objet tridimensionnel.

10. Film selon la revendication 8,
dans lequel les une ou plusieurs régions porteuses comprennent une pluralité de régions porteuses, chaque région porteuse, individuellement, comprenant un ou plusieurs agents bioactifs, les un ou plusieurs agents bioactifs étant identiques ou différents, et dans lequel chacune de la pluralité de régions porteuses, individuellement, est positionné sur l'une et/ou l'autre des première et seconde faces du film ; et
dans lequel le film comprend en outre une pluralité de régions vierges, chaque région vierge comprenant une couche qui ne contient pas d'adhésif ni d'agent bioactif, les une ou plusieurs régions vierges étant positionnées sur la première face ou la seconde face de la couche de film et entre ou de manière adjacente à l'une de la pluralité de régions porteuses ou des régions adhésives.

11. Film selon la revendication 10, dans lequel au moins l'une de la pluralité de régions porteuses est positionnée sur le film de telle sorte qu'elle se trouve directement au-dessus d'une autre de la pluralité de régions porteuses lorsque le film est enroulé autour de l'objet tridimensionnel, ou
dans lequel au moins l'une de la pluralité de régions vierges est positionnée sur le film de telle sorte qu'elle se trouve directement au-dessus de l'une de la pluralité de régions porteuses lorsque le film est enroulé autour de l'objet tridimensionnel et la région vierge agit comme un régulateur pour la libération de l'agent bioactif, ou
dans lequel au moins l'une de la pluralité de régions vierges agit comme un régulateur pour la libération de l'agent bioactif lorsque le film est enroulé autour de l'objet tridimensionnel.

12. Dispositif, tissu ou structure anatomique présentant un film auto-adhésif selon la revendication 1 pour l'administration d'un ou plusieurs agents bioactifs dans un corps, le dispositif, le tissu ou la structure anatomique comprenant :
un dispositif, un tissu ou un élément de structure anatomique présentant une surface externe et une forme sensiblement tridimensionnelle ; et
un film auto-adhésif selon la revendication 1.

13. Dispositif, tissu ou structure anatomique selon la revendication 12, dans lequel/laquelle le dispositif, le tissu ou la structure anatomique est un dispositif sélectionné dans le groupe consistant en des cathéters, des cathéters vasculaires, des greffons vasculaires, des greffons artérioveineux, des cathéters urinaires, des endoprothèses urinaires, des cathéters sus-pubiens, des tubes trachéaux, des sondes d'oesophagostomie, une prothèse vocale trachéo-oesophagienne, des cathéters de dialyse péritonéale, des drains/sondes de drainage, des tubes de tympanostomie, des implants orthopédiques, des dispositifs de fixation orthopédique, des stimulateurs cardiaques, des dispositifs d'assistance cardiaque, des dispositifs de neurostimulation, des dispositifs d'administration de médicaments et des sondes d'alimentation.

14. Dispositif, tissu ou structure anatomique selon la revendication 12, dans lequel/laquelle le dispositif, le tissu ou la structure anatomique est un tissu ou une structure anatomique sélectionné(e) dans le groupe consistant en les nerfs, les vaisseaux, les tissus conjonctifs incluant les tendons, les ligaments et les os, l'œsophage, l'intestin, le côlon, les anastomoses chirurgicales et les fistules.
